# EUROPEAN PATENT APPLICATION

(11) **EP 4 631 511 A1**
(43) Date of publication of application: **15.10.2025**
(21) Application number: 24774198.6
(22) Date of filing: 21.03.2024
(51) Int. Cl.: A61K 35/747, C12N 1/20

(54) **PROBIOTIC AND USE THEREOF**

(30) Priority: 21.03.2023 CN 202310272535; 16.05.2023 CN 202310548421; 16.05.2023 CN 202310548318; 16.05.2023 CN 202310548072; 19.05.2023 CN 202310568015
(71) Applicant: Sichuan Anaerobic Biotechnology Co., Ltd., Chengdu, Sichuan 610219 (CN)
(72) Inventor: LI, Jingxin, Chengdu, Sichuan 610219 (CN); ZHANG, Xufei, Chengdu, Sichuan 610219 (CN); FU, Fang, Chengdu, Sichuan 610219 (CN); CUI, Yaqian, Chengdu, Sichuan 610219 (CN); JIN, Meiyu, Chengdu, Sichuan 610219 (CN); XIANG, Chen, Chengdu, Sichuan 610219 (CN); XIAO, Yuanling, Chengdu, Sichuan 610219 (CN); CHEN, Xin, Chengdu, Sichuan 610219 (CN); SHUI, Junrui, Chengdu, Sichuan 610219 (CN)
(74) Representative: karo IP
(86) International application number: PCT/CN2024/082932
(87) International publication number: WO 2024/193633

(57) **Abstract**

Disclosed in the present invention is a probiotic. The probiotic comprises Parabacteroides distasonis with the microbial deposit number of CCTCC NO: M20222033, Limosilactobacillus fermentum with the microbial deposit number of CCTCC NO: M2023352, Lactobacillus salivarius with the microbial deposit number of CCTCC NO: M2023348, Enterococcus avium with the microbial deposit number of CCTCC NO: M2023350, and Bifidobacterium bifidum with the microbial deposit number of CCTCC NO: M2023349. The probiotic has good safety, has antioxidant activity, can inhibit pathogenic bacteria, can alleviate colon injury, can inhibit inflammation, and can alleviate diarrhea caused by chemotherapeutic drugs.

## Description

### TECHNICAL FIELD

The present disclosure relates to the technical field of microorganisms, and in particular to a probiotic and use thereof.

### BACKGROUND

Intestinal microorganisms are closely related to human health and are vividly referred to as "microbial organs". Intestinal flora is an important constituent of the body. Under normal conditions, the intestinal flora maintains dynamic stability. The intestinal microorganisms play an important role in promoting the digestion and absorption of nutrients, maintaining normal physiological functions of the intestinal tract, regulating immunity in the body, and other life activities. However, the intestinal flora is susceptible to numerous factors such as environmental factors, diet and lifestyle, mental factors, disease states, tumor treatment, use of antibiotics, and age. When the human intestinal flora is affected by the above factors, intestinal disorders (intestinal dysbacteriosis) may occur. The intestinal disorders may be manifested as diseases such as loss of intestinal beneficial bacteria, excessive reproduction of intestinal pathogenic bacteria, impaired intestinal barrier function, and intestinal inflammation. The intestinal disorders may further cause constipation, diarrhea, abdominal pain, abdominal distension, and other gastrointestinal diseases in hosts. Severe intestinal disorders can progress to diseases such as inflammatory bowel disease, ulcerative colitis, and irritable bowel syndrome. The aforementioned diseases greatly affect human health and quality of life.

Currently, using intestinal probiotics to improve intestinal health and prevent or treat intestinal diseases is of increasing interest. The intestinal probiotics can enhance the intestinal mucosal barrier function, prevent the adhesion and colonization of pathogenic bacteria, enhance the immune response of the intestinal system, and the like, thereby achieving the effect of maintaining intestinal health. For example, the Chinese patent application CN 102711778A discloses a strain of *Bifidobacterium animalis subsp. lactis* DN-173010, and verifies that its fermented milk is capable of alleviating ulcerative colitis through mouse experiments and histological studies. The patent application with Publication No. CN107312726A discloses *Lactobacillus plantarum,* which can inhibit the growth of harmful bacteria such as *Escherichia coli, Salmonella, Streptococcus suis,* and *Staphylococcus aureus* in the intestinal tract.

The probiotics are also used to prevent or ameliorate side effects of flora disorders caused by some drugs such as antibiotics. Clinically, the side effects associated with chemoradiotherapy are common. The diarrhea caused by chemotherapeutic drugs, also known as chemotherapy-induced diarrhea (CID), is one of the most common complications during chemotherapy for tumor patients. The development of CID is considered to be multifactorial. However, the pathogenesis of CID is not well defined. In the case of 5-fluorouracil (5-FU), proliferating small intestinal cells are relatively sensitive to 5-FdUMP or 5-FUMP produced by the phosphorylation of 5-FU. 5-FdUMP or 5-FUMP can cause damage to the mucosa of the small intestine and interfere with the division of intestinal cells, thereby resulting in necrosis of intestinal wall cells and extensive inflammation in the intestinal wall, leading to an imbalance in the number of absorbed and secreted cells in the small intestine, and further causing diarrhea. In addition, chemotherapeutic drugs can also cause cellular DNA damage and mitochondrial dysfunction, thereby resulting in ROS production and cell apoptosis. ROS can activate NF-κB and further up-regulate the expression of pro-inflammatory factors, thereby leading to the damage to intestinal epithelium, endothelium, and connective tissue. In the case of the damage to the intestinal epithelium, harmful bacteria are prone to colonization, and the intestinal microecology is damaged, thereby causing pathogenic bacterial infections and promoting the development and progression of diarrhea.

There is still a lack of unified and effective treatment means for CID clinically. Overall, the treatment for CID mainly aims to control symptoms, alleviate patients' pain, accelerate mucosal repair, and prevent secondary infections. The typical treatment for CID includes the use of antidiarrheal drugs, mucosal protective drugs, and antibacterial drugs, as well as high-dose use of loperamide and even the somatostatin drug octreotide. However, the above drugs have a single mechanism of action and significant side effects, and are not suitable for long-term maintenance medication. For example, the high-dose use of loperamide may cause the risk of paralytic ileus; the use of octreotide may cause side effects such as gallstones, hyperglycemia, and impaired glucose tolerance; and antibacterial drugs may further kill intestinal beneficial bacteria and disrupt the flora structure, thereby leading to intestinal microecological disorders.

The intestinal flora with high abundance and high diversity is a complex microbial ecosystem, which is an important natural barrier against intestinal pathogens and other risk factors. M. Kverka et al. (Clinical and Experimental Immunology, 163: 250-259, 2011) reported that *Parabacteroides distasonis* is able to ameliorate DSS-induced colitis in mice. The patent CN113215063B discloses *Lactobacillus salivarius* CPU-1, which can alleviate toxic and side effects caused by the chemotherapeutic drug temozolomide and focuses on the amelioration of the symptoms of mucositis by *Lactobacillus salivarius* CPU-1. Patents such as CN1511945A and CN86103736A have studied the use of various probiotics, including *Lactobacillus fermentum*, in the treatment or prevention of diarrhea. The patent CN113234619B discloses a *Bifidobacterium bifidum* strain capable of alleviating acute intestinal injury. An inactivated *Bacteroides fragilis* ZY-312 has been reported to be approved by the FDA for conducting CID clinical trials.

Although some studies have explored the possibility of using probiotics to prevent or treat a variety of intestinal diseases, there is still a lack of clinically effective and safe probiotic intervention regimens (particularly interventions for diseases or symptoms such as CID that lack effective treatment means). The development of a novel probiotic to overcome the defects of existing drugs such as high toxic and side effects and a single treatment mechanism, and to provide an effective treatment regimen for patients with cancer treatment-related diarrhea is still an urgent issue to be addressed in the field of medical biology.

### SUMMARY

In view of the deficiencies in the prior art, the first aspect of the present disclosure provides a probiotic composition, wherein the active ingredient in the probiotic composition comprises any one or a combination of two or more of the following: *Bifidobacterium bifidum* with the microbial deposit number of CCTCC NO: M 2023349 or a pure culture thereof, *Enterococcus avium* with the microbial deposit number of CCTCC NO: M 2023350 or a pure culture thereof, *Lactobacillus salivarius* with the microbial deposit number of CCTCC NO: M 2023348 or a pure culture thereof, *Limosilactobacillus fermentum* with the microbial deposit number of CCTCC NO: M 2023352 or a pure culture thereof, and *Parabacteroides distasonis* with the microbial deposit number of CCTCC NO: M 20222033 or a pure culture thereof.

In some embodiments, the probiotic composition further comprises a lyoprotectant, a food material, a pharmaceutically acceptable carrier, and/or a pharmaceutically acceptable excipient.

The second aspect of the present disclosure provides an isolated *Parabacteroides distasonis* strain or a pure culture thereof, wherein the *Parabacteroides distasonis* strain has a microbial deposit number of CCTCC NO: M20222033.

The third aspect of the present disclosure provides an isolated *Limosilactobacillus fermentum* strain or a pure culture thereof, wherein the *Limosilactobacillus fermentum* strain has a microbial deposit number of CCTCC NO: M2023352.

The fourth aspect of the present disclosure provides an isolated *Lactobacillus salivarius* strain or a pure culture thereof, wherein the *Lactobacillus salivarius* strain has a microbial deposit number of CCTCC NO: M2023348.

The fifth aspect of the present disclosure provides an isolated *Enterococcus avium* strain or a pure culture thereof, wherein the *Enterococcus avium* strain has a microbial deposit number of CCTCC NO: M2023350.

The sixth aspect of the present disclosure provides an isolated *Bifidobacterium bifidum* strain or a pure culture thereof, wherein the *Bifidobacterium bifidum* strain has a microbial deposit number of CCTCC NO: M2023349.

The seventh aspect the present disclosure provides a method for preventing, treating, or slowing an intestinal disease progression, which comprises administering to a subject a therapeutically effective amount of the probiotic composition according to the first aspect of the present disclosure, or the strain or the pure culture thereof according to any one of the second to sixth aspects of the present disclosure.

In some embodiments, the subject is a mammal; further preferably, the subject is a human.

In some embodiments, the probiotic composition according to the first aspect of the present disclosure, or the strain or the pure culture thereof according to any one of the second aspect, the third aspect, the fourth aspect, the fifth aspect, and the sixth aspect of the present disclosure, inhibits intestinal pathogenic bacteria in the subject, ameliorates colon injury of the subject, suppresses intestinal inflammation of the subject, repairs intestinal barrier of the subject, or alleviates symptoms of diarrhea in the subject.

In some embodiments, the intestinal disease is selected from an infection caused by an intestinal pathogenic bacterium, diarrhea, and radiation enteritis; preferably, the intestinal pathogenic bacterium is selected from one or more of *Pseudomonas aeruginosa, Salmonella* Paratyphi B, *Yersinia enterocolitica, Staphylococcus aureus, Vibrio parahaemolyticus, Clostridioides difficile*, *Shigella,* and *Escherichia coli*; preferably, the diarrhea is diarrhea caused by an anti-tumor drug, and further preferably, the diarrhea caused by an anti-tumor drug is diarrhea caused by a chemotherapeutic drug.

In some embodiments, the diarrhea caused by a chemotherapeutic drug is diarrhea caused by one or more drugs selected from the following: epirubicin, actinomycin D, doxorubicin, daunorubicin, paclitaxel, docetaxel, albumin-bound paclitaxel, cisplatin, carboplatin, nedaplatin, oxaliplatin, lobaplatin, cyclophosphamide, nitrogen mustard, carmustine, camptothecin, hydroxycamptothecin, topotecan, irinotecan, capecitabine, gemcitabine, methotrexate, 5-fluorouracil, pemetrexed, and cytarabine.

### BRIEF DESCRIPTION OF THE DRAWINGS

FIG. 1 shows top-view photographs illustrating the colony morphology of 5 strains.
FIG. 2 shows the experimental results of the total antioxidant capacity of 5 strains.
FIG. 3 shows the results of bacteriostatic experiments of 5 strains.
FIG. 4 shows the test results of *Lactobacillus salivarius* Lsali-1 and *Enterococcus avium* Eaviu-1 on the barrier repair of Caco-2 cells.
FIG. 5 shows the results of *in vitro* cell inflammation inhibition tests of 5 strains.
FIG. 6 shows the detection results of the adhesion ability of 5 strains to Caco-2 cells.
FIG. 7 shows the improvement of the diarrhea scores and total diarrhea score by *Parabacteroides distasonis* Pdist-1 in a mouse model with 5-fluorouracil-induced diarrhea.
FIG. 8 shows the improvement of the diarrhea scores and total diarrhea score by *Limosilactobacillus fermentum* Lferm-1 in a mouse model with 5-fluorouracil-induced diarrhea.
FIG. 9 shows the improvement of the diarrhea scores and total diarrhea score by *Lactobacillus salivarius* Lsali-1 in a mouse model with 5-fluorouracil-induced diarrhea.
FIG. 10 shows the improvement of the diarrhea scores and total diarrhea score by *Enterococcus avium* Eaviu-1 in a mouse model with 5-fluorouracil-induced diarrhea.
FIG. 11 shows the improvement of the diarrhea scores and total diarrhea score by *Bifidobacterium bifidum* Bbifi-1 in a mouse model with 5-fluorouracil-induced diarrhea.
FIG. 12 shows the improvement of the diarrhea scores and total diarrhea scores by *Bifidobacterium bifidum* strains Bbifi-1 and DSM20456 in mouse models with 5-fluorouracil-induced diarrhea.
FIG. 13 shows histopathological results of mice with 5-fluorouracil-induced diarrhea treated with *Parabacteroides distasonis* Pdist-1 and *Limosilactobacillus fermentum* Lferm-1, with a scale bar of 500 µm.
FIG. 14 shows the effects of 4 strains on the relative expression levels of colonic inflammatory factors and the aquaporin gene in mice with 5-fluorouracil-induced diarrhea.
FIG. 15 shows the therapeutic effect of the *Bifidobacterium bifidum* strain Bbifi-1 on mice with radiation enteritis-induced diarrhea.
FIG. 16 shows photographs illustrating co-culture characteristics of five strains on BF839 agar media.

### DETAILED DESCRIPTION

In order to make the purposes, technical solutions, and advantages of the present disclosure clearer, the embodiments of the present disclosure will be further described in detail with reference to the accompanying drawings.

The present disclosure provides a probiotic composition for preventing, treating, or slowing an intestinal disease, wherein an active ingredient of the probiotic composition comprises a therapeutically effective amount of any one or a combination of two or more selected from the following: *Bifidobacterium bifidum* with the microbial deposit number of CCTCC NO: M 2023349 or a pure culture thereof, *Enterococcus avium* with the microbial deposit number of CCTCC NO: M 2023350 or a pure culture thereof, *Lactobacillus salivarius* with the microbial deposit number of CCTCC NO: M 2023348 or a pure culture thereof, *Limosilactobacillus fermentum* with the microbial deposit number of CCTCC NO: M 2023352 or a pure culture thereof, and *Parabacteroides distasonis* with the microbial deposit number of CCTCC NO: M 20222033 or a pure culture thereof. The 5 strains with the specific microbial deposit numbers claimed in the present disclosure include, but are not limited to: (1) strains with the specific microbial deposit numbers deposited in the depository; (2) strains having the same genomes as the strains in (1); (3) passaged strains without gene mutations based on the aforementioned (1) or (2); (4) passaged strains that accumulate minor mutations during passaging, but have no substantial changes in toxicity, immunogenicity, and bioactivity based on the aforementioned (1), (2), or (3); (5) live bacteria, inactivated products of the live bacteria, lysates of the live bacteria, or fermentation products of the live bacteria based on any one of the aforementioned strains (1) to (4).

The strains having the same genome include, but are not limited to, strains having the same genetic background independently isolated and disclosed by others after the corresponding priority date of the present disclosure, i.e., strains isolated from nature or animals (including humans) having the same genome (the same genetic background). Conventional cultures are generally considered to be passaged strains without gene mutations. As is known in the art, minor mutations are generally inevitably introduced when strains are passaged. When the mutation occurs in a non-coding sequence region, or is a synonymous mutation in a coding region, or is a mutation that does not affect the toxicity, immunogenicity, and bioactivity of the strains (e.g., it may be a linker amino acid residue between two domains, or a residue of a minor mutation located within the higher-order structure of a protein that does not affect the toxicity, immunogenicity, and bioactivity due to the absence of contact with immune cells), it can be reasonably expected that the purposes of the present disclosure can still be achieved if these minor changes do not significantly affect the toxicity, immunogenicity, and bioactivity of the progeny strains, and these minor changes are derived from the strains contributed by the present disclosure and thus still fall within the substantial technical contribution of the present disclosure. These minor mutations are still insubstantial mutations, and the strains with these minor mutations should be considered as mutant strains that have no changes in toxicity, immunogenicity, and bioactivity. In terms of detection, there is no substantial change in toxicity, immunogenicity, and bioactivity, including but not limited to, being the same in toxicity, immunogenicity, and bioactivity within the limitations of detection sensitivity, limit of detection, and other detection techniques and within acceptable or inevitable errors. The toxicity, immunogenicity, and bioactivity of the progeny of the strains are determined using cells, animals, etc., and there is no substantial change due to differences in cell lines, animal species, age, sex, health status, culture conditions, and the like, as well as expected or inevitable systematic errors. The active ingredient refers to a substance that functions as a component to produce a biological effect. In the present disclosure, the active ingredient is a probiotic strain. Through the research on the co-culture characteristics of the five strains in the present disclosure, it can be found that these strains do not inhibit each other. Therefore, compositions containing 2, 3, 4, or 5 strains can be formulated according to the efficacy characteristics of these strains, and it is reasonably expected that the compositions formulated with these strains can simultaneously exert the efficacy of the strains in the group.

In order to better illustrate a suitable combination of the strains in the present disclosure, it is proposed that M1 represents a *Parabacteroides distasonis* strain with the microbial deposit number of CCTCC NO: M20222033 or a pure culture thereof; M2 represents an *Enterococcus avium* strain with the microbial deposit number of CCTCC NO: M2023350 or a pure culture thereof; M3 represents a *Limosilactobacillus fermentum* strain with the microbial deposit number of CCTCC NO: M2023352 or a pure culture thereof; M4 represents a *Bifidobacterium bifidum* strain with the microbial deposit number of CCTCC NO: M2023349 or a pure culture thereof; and M5 represents a *Lactobacillus salivarius* strain with the microbial deposit number of CCTCC NO: M2023348 or a pure culture thereof.

In some embodiments, the active ingredient in the probiotic composition of the present disclosure is any one, any two, any three, any four, or five of M1, M2, M3, M4, and M5.

In some embodiments, the active ingredient in the probiotic composition is a combination of M1 and any one, any two, or any three selected from M2, M3, M4, and M5.

In some embodiments, the active ingredient in the probiotic composition is a combination of M2 and any one, any two, or any three selected from M1, M3, M4, and M5.

In some embodiments, the active ingredient in the probiotic composition is a combination of M3 and any one, any two, or any three selected from M1, M2, M4, and M5.

In some embodiments, the active ingredient in the probiotic composition is a combination of M4 and any one, any two, or any three selected from M1, M2, M3, and M5.

In some embodiments, the active ingredient in the probiotic composition is a combination of M5 and any one, any two, or any three selected from M1, M2, M3, and M4.

In some embodiments, the active ingredient is a strain of a single species, and the tests have shown that even strains containing only a single species can play a significant role in preventing, treating, or slowing an intestinal disease.

Therefore, in some embodiments, the present disclosure provides use of *Parabacteroides distasonis* with the microbial deposit number of CCTCC NO: M20222033 or a pure culture thereof in the preparation of a formulation for preventing, slowing, or treating a disease or sub-health status, wherein preventing, slowing, or treating the disease or sub-health status comprises: preventing, treating, or slowing oxidative damage in the intestinal tract; inhibiting any one, any two, any three, any four, or five of *Pseudomonas aeruginosa, Salmonella* Paratyphi B, *Yersinia enterocolitica, Staphylococcus aureus,* and *Vibrio parahaemolyticus* in the intestinal tract; increasing the expression level of AQP8; and tolerating, resisting, preventing, or alleviating diarrhea.

In some embodiments, the diarrhea is selected from diarrhea caused by a chemotherapeutic drug.

In some embodiments, the chemotherapeutic drug is selected from 5-fluorouracil.

In some embodiments, the present disclosure provides use of *Limosilactobacillus fermentum* with the microbial deposit number of CCTCC NO: M2023352 or a passaged bacterium thereof in the preparation of a formulation for preventing a disease or sub-health status, slowing a disease or sub-health status, or treating a disease or sub-health status, wherein preventing the disease or sub-health status, slowing the disease or sub-health status, or treating the disease or sub-health status comprises: preventing, treating, or slowing oxidative damage in the intestinal tract; inhibiting any one, any two, any three, or four of *Salmonella* Paratyphi B, *Yersinia enterocolitica, Staphylococcus aureus,* and *Clostridioides difficile* in the intestinal tract; reducing the expression level of TNF-α; reducing the expression level of IL-6; and tolerating, resisting, preventing, or alleviating diarrhea.

In some embodiments, the diarrhea is selected from diarrhea caused by a chemotherapeutic drug.

In some embodiments, the chemotherapeutic drug is selected from 5-fluorouracil.

In some embodiments, the present disclosure provides use of *Lactobacillus salivarius* with the microbial deposit number of CCTCC NO: M2023348 or a passaged bacterium thereof in the preparation of a formulation for preventing a disease or sub-health status, slowing a disease or sub-health status, or treating a disease or sub-health status, wherein preventing the disease or sub-health status, slowing the disease or sub-health status, or treating the disease or sub-health status comprises: preventing, treating, or slowing oxidative damage in the intestinal tract; inhibiting any one, any two, any three, any four, any five, any six, or seven of *Pseudomonas aeruginosa, Shigella, Salmonella* Paratyphi B, *Yersinia enterocolitica, Vibrio parahaemolyticus, Staphylococcus aureus,* and *Clostridioides difficile* in the intestinal tract; preventing, ameliorating, or repairing intestinal barrier damage caused by inflammation; reducing the expression level of TNF-α; reducing the expression level of IL-6; reducing the expression level of IL-1β; and tolerating, resisting, preventing, or alleviating diarrhea.

In some embodiments, the diarrhea is selected from diarrhea caused by a chemotherapeutic drug.

In some embodiments, the present disclosure provides use of *Enterococcus avium* with the microbial deposit number of CCTCC NO: M2023350 or a passaged bacterium thereof in the preparation of a formulation for preventing a disease or sub-health status, slowing a disease or sub-health status, or treating a disease or sub-health status, wherein preventing the disease or sub-health status, slowing the disease or sub-health status, or treating the disease or sub-health status comprises: preventing, treating, or slowing oxidative damage in the intestinal tract; inhibiting any one or two of *Pseudomonas aeruginosa* and *Clostridioides difficile* in the intestinal tract; preventing, ameliorating, or repairing intestinal barrier damage caused by inflammation; reducing the expression level of TNF-α; reducing the expression level of IL-6; and tolerating, resisting, preventing, or alleviating diarrhea.

In some embodiments, the diarrhea is selected from diarrhea caused by a chemotherapeutic drug.

In some embodiments, the chemotherapeutic drug is selected from 5-fluorouracil.

In some embodiments, the present disclosure provides use of *Bifidobacterium bifidum* with the microbial deposit number of CCTCC NO: M2023349 or a passaged bacterium thereof in the preparation of a formulation for preventing a disease or sub-health status, slowing a disease or sub-health status, or treating a disease or sub-health status, wherein preventing the disease or sub-health status, slowing the disease or sub-health status, or treating the disease or sub-health status comprises: preventing, treating, or slowing oxidative damage in the intestinal tract; inhibiting any one, any two, any three, any four, any five, any six, or seven of *Pseudomonas aeruginosa, Shigella, Escherichia coli, Salmonella* Paratyphi B, *Yersinia enterocolitica, Vibrio parahaemolyticus,* and *Clostridioides difficile* in the intestinal tract; reducing the expression level of TNF-α; and tolerating, resisting, preventing, or alleviating diarrhea.

In some embodiments, the diarrhea is selected from diarrhea caused by a chemotherapeutic drug and diarrhea caused by radiation enteritis.

In some embodiments, the chemotherapeutic drug is selected from 5-fluorouracil.

In some embodiments, the radiation enteritis is radiation enteritis caused by abdominal X-ray irradiation.

The therapeutically effective amount refers to the amount of an active ingredient effective to prevent, treat, alleviate, or ameliorate symptoms or progression of a disorder (e.g., intestinal diseases: diarrhea, infection, inflammation, etc.) or to prolong the survival time of a subject to be treated. Determination of the therapeutically effective amount is undoubtedly within the ability of those skilled in the art, especially in light of the detailed disclosure provided herein. The therapeutically effective amount or dose may be estimated initially from *in vitro* and cell culture assays, and the dose is formulated in animal models to achieve a desired concentration or potency. Such information may be used to more accurately determine useful doses for humans.

In some embodiments, the probiotic composition administered to a subject at a single dose contains 10²-10¹⁵ CFU, 10³-10¹⁴ CFU, 10⁴-10¹³ CFU, 10⁵-10¹² CFU, 10⁶-10¹² CFU, 10⁷-10¹¹ CFU, or 10⁸-10¹⁰ CFU of probiotics. The dose may vary depending on the dosage form used and the route of administration used. The exact dose may be selected by individual physicians according to the condition of patients. The dose and interval may be adjusted individually to provide a sufficient amount of the active ingredient to induce a biological effect. The administration may be performed at a single dose or multiple doses depending on the severity and responsiveness of the disorder to be treated, with the course of treatment lasting from several days to several weeks or until a cure or alleviation of the disease state is achieved. Primarily, the amount of the composition to be administered depends on the subject to be treated, the severity of the disease, the route of administration, the judgment of the prescribing physician, and the like.

The present disclosure provides a composition in the form of a common food, a beverage, a healthcare product, a medical food, or a pharmaceutical product, which comprises the probiotic composition or the strain described in the present disclosure. These common foods, beverages, healthcare products, medical foods, or pharmaceutical products comprise various exemplary embodiments of the composition of the present disclosure. These common foods, beverages, healthcare products, medical foods, or pharmaceutical products may be prepared into or provided as probiotic powders, capsules, grains, baby foods, health foods, or foods for specific health uses, and may also be pharmaceutical capsules, tablets, powders, and the like. The probiotic composition of the present disclosure may further comprise other beneficial active ingredients, such as an additional probiotic with antidiarrheal function, prebiotics, or drugs. The prebiotics help to regulate the intestinal environment by promoting the growth of probiotics in the intestinal tract, thereby indirectly exerting an antidiarrheal effect. Examples of the second beneficial active ingredients include, but are not limited to, *Bacillus licheniformis, Bifidobacterium, Clostridium butyricum,* fructo-oligosaccharide, galacto-oligosaccharide, isomalto-oligosaccharide, xylo-oligosaccharide, mannan oligosaccharide, inulin, stachyose, soybean oligosaccharide, β-glucan, lactosucrose, and the like.

"Any one" refers to optionally selecting a single option from the provided options. "Two or more" refers to optionally selecting two, three, ..., or up to all available options from the provided options as selection schemes.

The intestinal disease refers to intestinal diseases such as infection, inflammation, diarrhea, or dysbacteriosis. In some embodiments, the intestinal disease refers to an infection caused by an intestinal pathogenic bacterium, colon injury, intestinal inflammation, intestinal barrier damage, and/or diarrhea.

In some embodiments, the intestinal disease is selected from an infection caused by an intestinal pathogenic bacterium, diarrhea, and radiation enteritis; preferably, the intestinal pathogenic bacterium is selected from one or more of *Pseudomonas aeruginosa, Salmonella* Paratyphi B, *Yersinia enterocolitica, Staphylococcus aureus, Vibrio parahaemolyticus, Clostridioides difficile*, *Shigella,* and *Escherichia coli*; preferably, the diarrhea is diarrhea caused by an anti-tumor drug, and further preferably, the diarrhea caused by an anti-tumor drug is diarrhea caused by a chemotherapeutic drug.

The intestinal pathogenic bacterium refers to those bacteria that are able to cause intestinal infections or diseases, which can enter the human body through contaminated food, water, direct contact, insect vectors, or the like, resulting in various intestinal diseases. There is a wide variety of intestinal pathogenic bacteria, and their survival and reproduction in the intestinal tract may disrupt the normal balance of flora in the intestinal tract, causing problems such as inflammation, diarrhea, and malabsorption.

In some embodiments, the intestinal pathogenic bacterium is selected from one or more of *Pseudomonas aeruginosa, Salmonella* Paratyphi B, *Yersinia enterocolitica, Staphylococcus aureus, Vibrio parahaemolyticus, Clostridioides difficile*, *Shigella,* and *Escherichia coli.* Radiation enteritis refers to an intestinal complication caused by malignant tumors in the pelvic cavity, abdominal cavity, and retroperitoneum after radiotherapy. This inflammation can affect the small intestine, colon, and rectum, and is therefore also known as radiation proctitis, colitis, and enteritis. The most common symptom of radiation enteritis is diarrhea, sometimes accompanied by mucus or bloody stools, and other symptoms also include abdominal pain, tenesmus, nausea and vomiting, abdominal distension, anorexia, and possibly weight loss. The diarrhea refers to a significantly increased frequency of bowel movements, usually exceeding 3 times a day, characterized by loose stools with high water content (exceeding 85%) and potentially accompanied by mucus, pus, blood, or undigested food. The causes of diarrhea include pathogenic bacterial infections, food poisoning, side effects of drugs, intestinal inflammation, psychological factors, and the like.

In some embodiments, the diarrhea is diarrhea caused by an anti-tumor drug. Diarrhea caused by anti-tumor drugs: The anti-tumor drugs may be broadly classified into cytotoxic drugs, small-molecule targeted drugs, monoclonal antibody drugs, immune checkpoint inhibitors, hormonal drugs, antibody-drug conjugates (ADCs), biological response modifiers, and other drugs according to the mechanism of action and the source of the drugs. The diarrhea caused by the use of these drugs is referred to as diarrhea caused by anti-tumor drugs, and also as cancer treatment-related diarrhea/tumor-associated diarrhea.

In some embodiments, the diarrhea caused by an anti-tumor drug refers specifically to diarrhea caused by a chemotherapeutic drug. Cytotoxic drugs are also commonly referred to as chemotherapeutic drugs. The mechanism of action of these drugs is mainly to inhibit or kill cancer cells by disrupting or interfering with the growth and division processes of tumor cells. Since chemotherapeutic drugs are usually highly toxic to rapidly dividing cells, they not only act on cancer cells, but also may affect rapidly dividing cells in normal body tissues, such as hair follicle cells, gastrointestinal cells, and bone marrow cells. These effects are common side effects of chemotherapy, such as alopecia, nausea, vomiting, diarrhea, and myelosuppression.

In some embodiments, the diarrhea caused by a chemotherapeutic drug is diarrhea caused by one or more drugs selected from the following: epirubicin, actinomycin D, doxorubicin, daunorubicin, paclitaxel, docetaxel, albumin-bound paclitaxel, cisplatin, carboplatin, nedaplatin, oxaliplatin, lobaplatin, cyclophosphamide, nitrogen mustard, carmustine, camptothecin, hydroxycamptothecin, topotecan, irinotecan, capecitabine, gemcitabine, methotrexate, 5-fluorouracil, pemetrexed, and cytarabine.

The present disclosure further provides a method for preventing, treating, or slowing an intestinal disease, which comprises administering to a subject the probiotic composition according to the first aspect of the present disclosure, or the strain or the pure culture thereof according to the second aspect of the present disclosure. The subject may be a poultry, a mammal, and a human, preferably a mammal and a human, and more preferably a human.

The present disclosure further provides 5 isolated probiotic strains for preventing or treating an intestinal disease, which are respectively: *Parabacteroides distasonis* or a progeny strain thereof, a clonal strain thereof, a fermentation product thereof, a lysate thereof, an extract thereof, and a pure culture thereof, the microbial deposit number of *Parabacteroides distasonis* being CCTCC NO: M20222033; *Limosilactobacillus fermentum* or a progeny strain thereof, a clonal strain thereof, a fermentation product thereof, a lysate thereof, an extract thereof, and a pure culture thereof, the microbial deposit number of *Limosilactobacillus fermentum* being CCTCC NO: M2023352; *Lactobacillus salivarius* or a progeny strain thereof, a clonal strain thereof, a fermentation product thereof, a lysate thereof, an extract thereof, and a pure culture thereof, the microbial deposit number of *Lactobacillus salivarius* being CCTCC NO: M 2023348; *Enterococcus avium* or a progeny strain thereof, a clonal strain thereof, a fermentation product thereof, a lysate thereof, an extract thereof, and a pure culture thereof, the microbial deposit number of *Enterococcus avium* being CCTCC NO: M2023350; and *Bifidobacterium bifidum* or a progeny strain thereof, a clonal strain thereof, a fermentation product thereof, a lysate thereof, an extract thereof, and a pure culture thereof, the microbial deposit number of *Bifidobacterium bifidum* being CCTCC NO: M2023349.

The technical solutions in the examples of the present disclosure will be described clearly and completely below. Apparently, the described examples are merely some rather than all of the examples of the present disclosure. Based on the examples of the present disclosure, all other examples obtained by those skilled in the art without creative efforts shall fall within the protection scope of the present disclosure. The materials and instruments not described in the present disclosure are conventional materials and instruments in the art. The operation details not described in the present disclosure are conventional operations in the art. The software used in the present disclosure is operated by conventional methods with reference to the instruction manual provided by the software provider. The kit used in the present disclosure is operated by conventional methods with reference to the instruction manual of the kit. Unless otherwise stated, the specific parameters in the present disclosure should be understood as being allowed to vary within a certain error range, such as ±5%. The temperature may fluctuate within the range of ±5 °C, ±4 °C, ±3 °C, ±2 °C, and ±1 °C.

The media used in the following examples are prepared as follows. Unless otherwise stated, the media may be prepared by methods commonly used in the art or may be commercially available.

Preparation of YCFA liquid medium: 10.0 g of peptone, 2.5 g of yeast extract, 0.45 mL of a 10% (w/w) aqueous MgSO₄·7H₂O solution, 0.45 mL of a 10 mg/mL aqueous CaCl₂ solution, 10 mL of TE141, 0.45 g of K₂HPO₄, 0.45 g of KH₂PO₄, 0.90 g of NaCl, and 3.2 mL of VFA-mix were added to 1 L of distilled water to obtain a solution. The solution was purged with N₂ to remove oxygen and aliquoted. The aliquoted solution was subjected to moist heat sterilization at a high temperature of 121 °C for 30 min for later use.

Preparation of TE141: 1.50 g of nitrilotriacetic acid was added to 200 mL of purified water to obtain a solution; an appropriate amount of NaOH was added to the solution until the solution became clear; 800 mL of water was added to the solution; and the pH value was adjusted to 5.5 with 50% HCl to obtain an aqueous nitrilotriacetic acid solution. 3.00 g of MgSO₄·7H₂O, 0.50 g of MnSO₄·H₂O, 1.00 g of NaCl, 0.10 g of FeSO₄·7H₂O, 0.18 g of CoSO₄·7H₂O, 0.10 g of CaCl₂·2H₂O, 0.18 g of ZnSO₄·7H₂O, 0.006 g of CuSO₄·5H₂O, 0.02 g of KAl(SO₄)₂·12H₂O, 0.01 g of H₃BO₃, 0.01 g of Na₂MoO₄·2H₂O, 0.03 g of NiCl₂·6H₂O, 0.03 mL of a 10 mg/mL Na₂SeO₃·5H₂O solution, and 0.03 mL of a 10 mg/mL Na₂WO₄·2H₂O solution were added to the above solution; and during the addition, the solution was stirred continuously to keep clear.

Preparation of VFA-mix: 90 mL of acetic acid, 30 mL of propionic acid, 10 mL of n-valeric acid, 10 mL of isobutyric acid, and 10 mL of butyric acid were mixed well to obtain a solution for later use. The pH was adjusted to neutrality with a 5 M NaOH solution before use.

Preparation of three-component mixed liquid medium (BHI + MRS + modified GAM): 19.25 g of BHI broth powder (Qingdao Hi-Tech Industrial Park Hope Bio-Technology Co., Ltd., HB8297-5), 13.5 g of MRS broth powder (Guangdong Huankai Biotechnology Co., Ltd., 027312), and 15 g of modified GAM broth powder (Qingdao Hi-Tech Industrial Park Hope Bio-Technology Co., Ltd., HB8518-3) were dissolved in 1 L of distilled water to obtain a solution. The solution was purged with N₂ to remove oxygen and aliquoted. The aliquoted solution was subjected to moist heat sterilization at a high temperature of 121 °C for 30 min.

Preparation of three-component mixed solid medium (BHI + MRS + modified GAM): 5 g of agar powder was added on the basis of the three-component mixed liquid medium, and the other steps were the same.

Preparation of two-component mixed medium (BHI + MRS): 19.25 g of BHI broth powder, 27.0 g of MRS broth powder, and 0.5 g of cysteine hydrochloride monohydrate were dissolved in 1 L of distilled water to obtain a mixed solution. Oxygen was removed, the mixed solution was aliquoted, and the aliquoted solution was subjected to moist heat sterilization at a high temperature of 121 °C for 15 min.

Preparation of BF839 medium: 6.0 g of potato extract powder (Beijing Solarbio Science & Technology Co., Ltd., FA0270), 10.0 g of multivalent peptone (Beijing Solarbio Science & Technology Co., Ltd., P8950-250), 5.0 g of proteose peptone (Qingdao Hi-Tech Industrial Park Hope Bio-Technology Co., Ltd., HB8277), 0.3 g of sodium thioglycolate, 5.0 g of yeast extract powder (Thermo Fisher Oxoid, LP0021B), 1.5 g of glucose, and 4.0 g of disodium hydrogen phosphate were dissolved in 1 L of distilled water to obtain a mixed solution. The mixed solution was purged with N₂ to remove oxygen and aliquoted. The aliquoted solution was subjected to moist heat sterilization at a high temperature of 121 °C for 30 min.

Preparation of MRS broth: 54.0 g of MRS broth powder and 0.5 g of cysteine hydrochloride monohydrate were weighed out and dissolved in 1 L of distilled water, and the mixed solution was purged with N₂ to remove oxygen and sterilized at 121 °C for 15 min.

Preparation of oxygen-free and resazurin-free PBS: 0.27 g of potassium dihydrogen phosphate, 1.42 g of disodium hydrogen phosphate, 8 g of sodium chloride, and 0.2 g of potassium chloride were dissolved in 1 L of distilled water. The mixed solution was heated to boiling and cooled to room temperature. 0.55 g of cysteine hydrochloride was added to the cooled solution, the resulting solution was stirred for dissolution, and then the pH was adjusted to 6.5. A quantitative liquid separator was connected. The mixed solution was purged with N₂, heated to boiling, and kept in a slightly boiling state for 30 min. After cooling, the solution was aliquoted at 400 mL/vial and subjected to moist heat sterilization at a high temperature of 121 °C for 30 min.

The solutions for the preparation of the MRS solid medium, GAM solid medium, TSB medium (tryptone soy broth, Qingdao Hi-Tech Industrial Park Hope Bio-Technology Co., Ltd., HB4114), TSAmedium(tryptone soy agar, Qingdao Hi-Tech Industrial Park Hope Bio-Technology Co., Ltd., HB4138), and brucella broth medium (Qingdao Hi-Tech Industrial Park Hope Bio-Technology Co., Ltd., HB0241) were all weighed out and dissolved according to the instructions, and then subjected to moist heat sterilization at a high temperature of 121 °C for 30 min.

Preparation of bacterial powder preparation medium of *Parabacteroides distasonis* Pdist-1: 6 g of anhydrous glucose, 15 g of soy peptone, 10 g of yeast extract powder, 10 g of yeast peptone, 2 g of potassium dihydrogen phosphate, 2 g of disodium hydrogen phosphate, 0.2 g of magnesium sulfate, 0.01 g of manganese sulfate, 0.2 g of calcium chloride, 1 mL of Tween 80, and 0.5 g of cysteine hydrochloride monohydrate were dissolved in 1 L of distilled water. The mixed solution was purged with N₂ to remove oxygen and aliquoted. The mixed solution was sterilized at 121 °C for 15 min.

Bacterial powder preparation medium of *Limosilactobacillus fermentum* Lferm-1: 30 g of anhydrous glucose, 15 g of soy peptone, 10 g of yeast extract powder, 5 g of sodium acetate, 2 g of potassium dihydrogen phosphate, 2 g of disodium hydrogen phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

Preparation of bacterial powder preparation medium of *Lactobacillus salivarius* Lsali-1: 24 g of anhydrous glucose, 20 g of soy peptone, 10 g of yeast extract powder, 10 g of peptone, 5 g of sodium acetate, 2 g of potassium dihydrogen phosphate, 2 g of disodium hydrogen phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

Bacterial powder preparation medium of *Enterococcus avium* Eaviu-1: 30 g of anhydrous glucose, 15 g of soy peptone, 10 g of yeast powder, 5 g of sodium acetate, 2 g of dipotassium phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

Bacterial powder preparation medium of *Bifidobacterium bifidum* Bbifi-1: 20 g of anhydrous glucose, 40 g of soy peptone, 5 g of N-acetylglucosamine, 2 g of potassium dihydrogen phosphate, 2 g of disodium hydrogen phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

Preparation of 0.1% Tween 80-PBS dilution: 3.58 g of disodium hydrogen phosphate dodecahydrate, 0.27 g of potassium dihydrogen phosphate, 8 g of sodium chloride, and 1 mL of Tween 80 were added to 1 L of boiling water and dissolved with a glass rod. 0.5 g of cysteine hydrochloride monohydrate was added to the aforementioned boiled solution. A Hungate apparatus was opened, and the solution was boiled again under N₂ atmosphere. After being purged with N₂ for 20 min, the solution was aliquoted into anaerobic bottles purged with N₂ for oxygen removal. The bottles were capped with stoppers and labeled, and the solutions were sterilized at a high temperature of 121 °C for 15 min.

### Example 1: Strain Isolation and Identification

Fresh stool samples were collected from several healthy human volunteers, and each stool sample was independently processed. An appropriate amount of oxygen-free PBS was added to the stool sample to obtain a mixture, and the mixture was shaken to obtain a suspension. The suspension was filtered with gauze under N₂ atmosphere to obtain a filtrate. The filtrate was centrifuged at 10,000 rpm for 20 min, then the supernatant was discarded, and the precipitate was retained. An appropriate amount of oxygen-free PBS was added to the precipitate to resuspend the bacteria, so as to obtain a suspension. An equal volume of a 50% (v/v) aqueous solution of oxygen-free glycerol was added to the suspension and mixed well to obtain a bacterial mixture sample. The sample was aliquoted into sample tubes, and the sample tubes were sealed in bags and vacuumized, and then stored in a refrigerator at -80 °C. The sample in each cryopreserved sample tube was thawed independently. 0.5 mL of the thawed sample was resuspended in 4.5 mL of oxygen-free PBS, and shaken and mixed well to obtain a bacterial suspension. Under anaerobic conditions, 0.5 mL of the bacterial suspension and 4.5 mL of anaerobic PBS were shaken and mixed well for dilution. The sample was serially diluted tenfold to a 10⁻⁶ dilution ratio using the same method. The bacterial solution with an appropriate dilution ratio was taken and well mixed with a YCFA liquid medium, the resulting mixture was then aliquoted into a 384-well plate, and the plate was anaerobically cultured at 37 °C for one week. The bacterial solution in a well where the bacteria had grown was inoculated into the YCFA medium and cultured for 48 h, and then the bacterial solution was aliquoted into two aliquots. An aliquot of the bacterial solution was examined by MALDI-TOF-MS for preliminary species classification of the isolated strain. After it was determined that the bacterial solution contained only bacteria (monoclonal strain) with one genetic background, another aliquot of the bacterial solution was inoculated into the YCFA medium again for culture according to the mass spectrometry result, where the 16S rDNA gene amplification and sequencing were performed on one aliquot; and a 50% (v/v) aqueous glycerol solution was added to another aliquot in a volume ratio of 1:1, and the mixture was well mixed and deposited.

The 16S rDNA gene sequence obtained by sequencing was aligned with the NCBI Nucleotide database to further identify the species of the isolated strain. From numerous strains for which the species were further determined, 5 strains were selected for further experiments of the present disclosure. Strain 1 has the highest sequence similarity to a strain of *Parabacteroides distasonis* (> 99%). Therefore, strain 1 was named *Parabacteroides distasonis* Pdist-1 (abbreviated as Pdist-1). Strain 2 has the highest sequence similarity to a strain of *Limosilactobacillus fermentum* (100%). Therefore, strain 2 was named *Limosilactobacillus fermentum* Lferm-1 (abbreviated as Lferm-1). Strain 3 has the highest sequence similarity to a strain of *Lactobacillus salivarius* (100.00%). Therefore, strain 3 was named *Lactobacillus salivarius* Lsali-1 (abbreviated as Lsali-1). Strain 4 has the highest sequence similarity to a strain of *Enterococcus avium* (100.00%). Therefore, strain 4 was named *Enterococcus avium* Eaviu-1 (abbreviated as Eaviu-1). Strain 5 has the highest sequence similarity to a strain of *Bifidobacterium bifidum* (99.86%). Therefore, strain 5 was named *Bifidobacterium bifidum* Bbifi-1 (abbreviated as Bbifi-1).

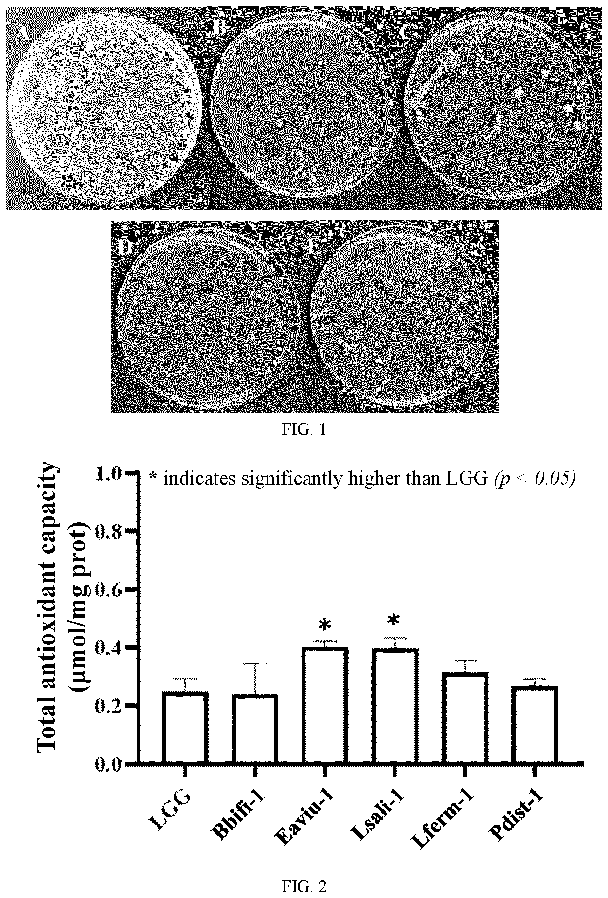

*Parabacteroides distasonis* Pdist-1, *Limosilactobacillus fermentum* Lferm-1, and *Enterococcus avium* Eaviu-1 were each inoculated into a BF839 medium and cultured to observe their colony morphology. *Lactobacillus salivarius* Lsali-1 and *Bifidobacterium bifidum* Bbifi-1 were each inoculated into a three-component mixed solid medium and cultured to observe their colony morphology. The top-view photographs illustrating the colony morphology of the aforementioned 5 strains are shown in FIG. 1, where A is a top-view photograph illustrating the colony morphology of *Parabacteroides distasonis* Pdist-1; B is a top-view photograph illustrating the colony morphology of *Limosilactobacillus fermentum* Lferm-1; C is a top-view photograph illustrating the colony morphology of *Lactobacillus salivarius* Lsali-1; D is a top-view photograph illustrating the colony morphology of *Enterococcus avium* Eaviu-1; and E is a top-view photograph illustrating the colony morphology of *Bifidobacterium bifidum* Bbifi-1. It can be seen that the 5 strains all exhibited white opaque round colonies with convex in the middle and smooth and moist surfaces.

### Example 2: Whole Genome Analysis of Strains

The 5 strains obtained in Example 1 were each inoculated into a three-component mixed liquid medium, and the bacteria were cultured until the late logarithmic growth phase was reached. The whole genome DNA of each strain was extracted and subjected to whole genome sequencing using the Illumina high-throughput sequencing platform NovaSeq 6000. After genome sequence assembly and annotation, the protein sequence was input into the virulence factor databases (VFDB) for virulence factor analysis. The results show that none of the 5 strains had virulence factors in their genomes.

The novelty analysis was performed on the 5 strains using the average nucleotide identity (ANI) method. Whole genome searches were performed in Genbank and the most similar strains were compared by fastANI (v1.33). The two strains with the highest whole genome similarity to *Parabacteroides distasonis* Pdist-1 are GCA_003462945.1 (ANI = 98.26%) and GCA_003459965.1 (ANI = 98.20%), respectively. The two strains with the highest whole genome similarity to *Limosilactobacillus fermentum* Lferm-1 are GCA_003465085.1 (ANI = 99.32%) and GCA_024385625.1 (ANI = 99.29%), respectively. The two strains with the highest whole genome similarity to *Lactobacillus salivarius* Lsali-1 are GCA_009863605.1 (ANI = 99.94%) and GCA_009866185.1 (ANI = 99.87%), respectively. The two strains with the highest whole genome similarity to *Enterococcus avium* Eaviu-1 are GCA_018917545.1 (ANI = 98.75%) and GCA_018373135.1 (ANI = 98.62%), respectively. The two strains with the highest whole genome similarity to *Bifidobacterium bifidum* Bbifi-1 are GCA_003466395.1 (ANI = 99.01%) and GCA_003437945.1 (ANI = 99.00%), respectively. It can be seen that the species classification in Example 1 was correct.

The whole genome sequences of the 5 strains were annotated by emapper-2.1.9, and it was found that the genome of *Parabacteroides distasonis* Pdist-1 has genes encoding 1 iso-bile acid-producing protein, 2 acetate-producing related enzymes, 3 propionate-producing related enzymes, 1 CAT (catalase)-producing related enzyme, and 1 SOD (superoxide dismutase)-producing related enzyme; the genome of *Limosilactobacillus fermentum* Lferm-1 has 1 gene encoding an acetate-producing related enzyme; the genome of *Lactobacillus salivarius* Lsali-1 has genes encoding 2 acetate-producing related enzymes and 1 propionate-producing related enzyme; the genome of *Enterococcus avium* Eaviu-1 has 2 genes encoding acetate-producing related enzymes, 1 gene encoding a propionate-producing related enzyme, 1 gene encoding SagA protein, and 1 gene encoding an SOD-related enzyme; and the genome of *Bifidobacterium bifidum* Bbifi-1 has genes encoding 1 acetate-producing related enzyme and 1 propionate-producing related enzyme.

The strains Pdist-1, Eaviu-1, Lferm-1, Bbifi-1, and Lsali-1 isolated and cultured in the present disclosure were each submitted to a depository recognized by the patent procedure and deposited. The depository is the China Center for Type Culture Collection (CCTCC); the address is Wuhan University, Wuhan, China; the culture names, classification and names, dates of deposit, dates of viability test, and microbial deposit numbers are shown in Table 1.

**Table 1. Summary of strain deposition information**

| Culture name | Classification and name | Date of deposit | Date of viability test | Microbial deposit number |
|---|---|---|---|---|
| *Parabacteroides distasonis* Pdist-1 | *Parabacteroides distasonis* | 2022-12-23 | 2022-12-30 | CCTCC NO:M20222033 |
| *Enterococcus avium* Eaviu-1 | *Enterococcus avium* | 2023-03-17 | 2023-03-24 | CCTCC NO:M2023350 |
| *Limosilactobacillus fermentum* Lferm-1 | *Limosilactobacillus fermentum* | 2023-03-17 | 2023-03-24 | CCTCC NO:M2023352 |
| *Bifidobacterium bifidum* Bbifi-1 | *Bifidobacterium bifidum* | 2023-03-17 | 2023-03-24 | CCTCC NO:M2023349 |
| *Lactobacillus salivarius* Lsali-1 | *Lactobacillus salivarius* | 2023-03-17 | 2023-03-24 | CCTCC NO:M2023348 |

### Example 3: Hemolysis Test

*Parabacteroides distasonis* Pdist-1 was inoculated into a three-component mixed liquid medium and anaerobically cultured at 37 °C for 12 h to obtain a bacterial solution containing an activated strain. *Enterococcus faecalis* (β-hemolysis, CICC23658, purchased from China Center of Industrial Culture Collection) was inoculated into a three-component mixed liquid medium and anaerobically cultured at 37 °C for 12 h to obtain a bacterial solution containing an activated strain (as a positive control). The three-component mixed liquid medium was used as a negative control. 2.5 µL of the two bacterial solutions containing the activated strains and the negative control were each inoculated into a Columbia blood agar plate (Shanghai Kemajia Microbe Technology Co., Ltd.), and 3 parallel tests were set for each sample. The Columbia blood agar plates were observed after 48 h of anaerobic culture at 37 °C. Results: A clearly defined and completely transparent hemolytic ring was formed around the *Enterococcus faecalis* colonies, indicating β-hemolysis; the medium around the *Parabacteroides distasonis* Pdist-1 colonies was unchanged, indicating γ-hemolysis, i.e., no hemolysis; the negative control had no hemolytic ring. The results show that *Parabacteroides distasonis* Pdist-1 has no hemolysis.

*Limosilactobacillus fermentum* Lferm-1, *Lactobacillus salivarius* Lsali-1, *Enterococcus avium* Eaviu-1, and *Bifidobacterium bifidum* Bbifi-1 were each inoculated into a three-component mixed liquid medium and anaerobically cultured at 37 °C until the late logarithmic growth phase was reached to obtain bacterial solutions containing the activated strains. 2 mL of 2% (v/v) fresh rabbit red blood cell PBS suspension (Beijing Bersee Science and Technology Co., Ltd.) was taken and subjected to liquid contact assay with the bacterial suspension resuspended in 2 mL of sterile normal saline. An equal volume of sterile water was used as a positive control, and an equal volume of sterile normal saline was used as a negative control. Each group was set in triplicate, and the samples were observed after being left to stand for 24 h. The results show that hemolysis occurred in the positive control; the negative control, *Limosilactobacillus fermentum* Lferm-1, *Lactobacillus salivarius* Lsali-1, *Enterococcus avium* Eaviu-1, and *Bifidobacterium bifidum* Bbifi-1 all had no hemolysis.

### Example 4: Antibiotic Susceptibility Test

According to the requirements for antibiotic susceptibility tests of anaerobic bacteria in "Technical specification on antimicrobial susceptibility tests" (Standard No. WS/T 639-2018) of the People's Republic of China Health Industry Standard, the susceptibility of the strains to antibiotics was determined by a broth dilution method, and the MIC values were recorded.

0.1 mL of the prepared 5 bacterial suspensions were sequentially pipetted into 96-well plates containing equal volumes of prepared antibacterial drugs, such that the final concentration of the inoculated bacteria was 5 × 10⁵ CFU/mL, and the drug concentrations were 256 µg/mL, 128 µg/mL, 64 µg/mL, 32 µg/mL, 16 µg/mL, 8 µg/mL, 4 µg/mL, 2 µg/mL, 1 µg/mL, 0.5 µg/mL, 0.25 µg/mL, and 0.125 µg/mL, respectively. Incubation: The above 96-well plates were anaerobically incubated in an incubator at 37 °C for 46-48 h. The results are shown in Table 2 below.

**Table 2. MIC values of 5 bacteria against 14 antibiotics (µg/mL)**

| Strain name | Pdist-1 | Lferm-1 | Lsali-1 | Eaviu-1 | Bbifi-1 |
|---|---|---|---|---|---|
| Penicillin | 8 | - | - | - | - |
| Ampicillin | 8 | 0.5 | 4 | 4 | 0.25 |
| Imipenem | 16 | - | - | - | - |
| Vancomycin | 8 | 0.125 | - | 2 | 1 |
| Ceftriaxone | 16 | - | - | - | - |
| Tetracycline | 8 | <0.125 | 0.5 | 0.5 | 0.125 |
| Erythromycin | 32 | <0.125 | 0.125 | 0.25 | 0.125 |
| Clindamycin | 16 | <0.125 | 8 | 4 | 1 |
| Levofloxacin | 8 | - | - | - | - |
| Gentamicin | <0.125 | 0.125 | 16 | 8 | 8 |
| Chloramphenicol | - | 0.125 | 256 | 128 | 8 |
| Kanamycin | - | - | 256 | - | - |
| Streptomycin | <0.125 | 0.125 | 128 | 128 | 128 |
| Rifampicin | <0.125 | - | - | - | - |

| | | | | | |
|---|---|---|---|---|---|
| Note: "-" indicates not tested. | | | | | |

### Example 5: Antioxidant Experiment

The activated 5 strains obtained in Example 1 were each inoculated into an oxygen-free BF839 medium. LGG (*Lactobacillus rhamnosus* GG, CICC6141, purchased from China Center of Industrial Culture Collection) was used as a positive control. The six strains were each subjected to the following procedures in parallel. All the strains were anaerobically cultured in the oxygen-free BF839 media at 37 °C for 24 h to obtain the cultured bacterial solutions.

0.5 mL of each of the cultured bacterial solutions was centrifuged at 12,000 rpm for 20 min, the supernatant was discarded, and the precipitate was resuspended in 0.5 mL of pre-cooled extract solution in a total antioxidant capacity assay kit for strains (the kit was purchased from Beijing Solarbio Science & Technology Co., Ltd., BC1315); the resuspension was transferred to a sterilized screw cap tube containing beads (purchased from Sigma, USA, G4649-1KG); the tube was shaken once for wall breaking by using a fast sample preparation instrument (parameter setting: 4.5 m/s, 30 s); the mixture was centrifuged at 12,000 rpm and 4 °C for 10 min; and the supernatant was placed on ice for later use. A BCA protein assay kit (the kit was purchased from Beijing Solarbio Science & Technology Co., Ltd., PC0020) was used, a standard curve was plotted according to the instruction of the kit, and a BCA sample was assayed. The antioxidant capacity of the sample was assayed using the total antioxidant capacity assay kit for strains (the kit was purchased from Beijing Solarbio Science & Technology Co., Ltd., BC1315) according to the instruction of the kit in combination with the standard curve. The unit of the total antioxidant capacity is µmol/mg prot. The experimental results are shown in FIG. 2. The total antioxidant capacities of *Lactobacillus salivarius* Lsali-1 and *Enterococcus avium* Eaviu-1 were significantly superior to that of the positive control strain LGG; the total antioxidant capacities of *Parabacteroides distasonis* Pdist-1, *Limosilactobacillus fermentum* Lferm-1, and *Bifidobacterium bifidum* Bbifi-1 were comparable to that of the positive control strain LGG. It can be seen that these strains all have relatively strong antioxidant capacities.

### Example 6: Test of Bacteriostatic Ability of Strains against Pathogenic Bacteria

The common intestinal pathogenic bacteria that can cause diarrhea as shown in Table 3 were selected to test the bacteriostatic ability of the 5 strains obtained in Example 1.

**Table 3. Pathogenic strain source information**

| Strain name | Microbial deposit number | Strain depository |
|---|---|---|
| *Pseudomonas aeruginosa* | CMCC(B)10104 | National Institutes for Food and Drug Control, PRC |
| *Salmonella* Paratyphi B | CMCC(B)50094 | National Institutes for Food and Drug Control, PRC |
| *Yersinia enterocolitica* | CMCC(B)52204 | National Institutes for Food and Drug Control, PRC |
| *Staphylococcus aureus* | CMCC(B)26003 | National Institutes for Food and Drug Control, PRC |
| *Vibrio parahaemolyticus* | ATCC 17802 | American Type Culture Collection |
| *Clostridioides difficile* | CICC 22951 | China Center of Industrial Culture Collection |
| *Shigella* | CMCC(B)51252 | National Institutes for Food and Drug Control, PRC |
| *Escherichia coli* | CMCC(B)44102 | National Institutes for Food and Drug |
| | | Control, PRC |

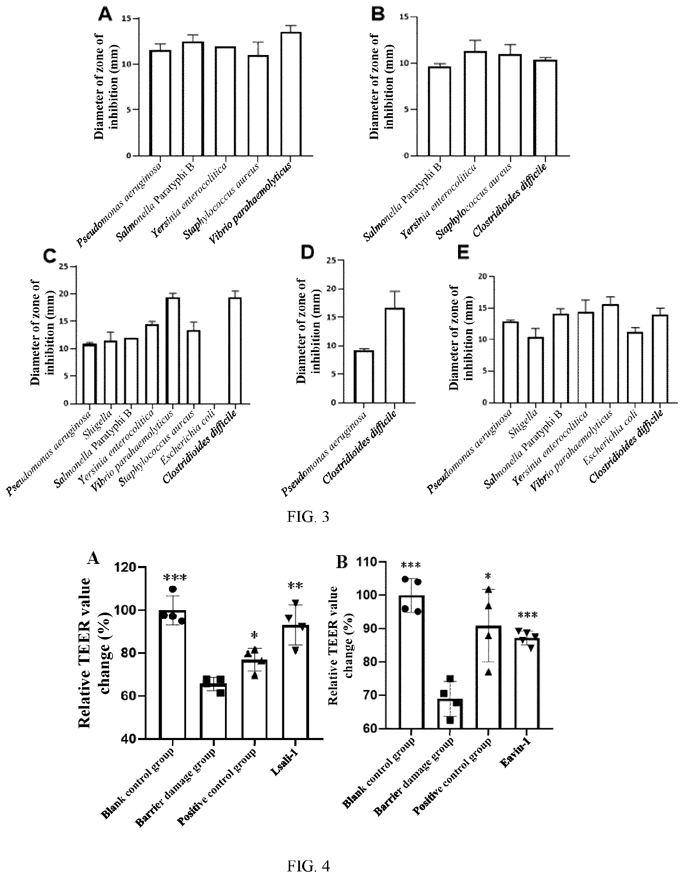

Preparation of strain fermentation broth: the activated 5 strains were each inoculated into a three-component mixed liquid medium and anaerobically cultured at 37 °C for 48 h to obtain fermentation broths. Preparation and spreading of pathogenic bacteria: *Salmonella* Paratyphi B, *Yersinia enterocolitica, Staphylococcus aureus, Pseudomonas aeruginosa, Shigella, Escherichia coli,* and *Vibrio parahaemolyticus* were each activated in a TSB broth medium (tryptone soy broth, Qingdao Hi-Tech Industrial Park Hope Bio-Technology Co., Ltd., HB4114), and then diluted to an appropriate concentration in a TSB broth medium; and 0.2 mL of the diluted bacterial solution was spread on a TSA solid medium (tryptone soy agar, Qingdao Hi-Tech Industrial Park Hope Bio-Technology Co., Ltd., HB4138). After anaerobic activation and transfer, *Clostridioides difficile* was diluted to an appropriate concentration in a three-component mixed liquid medium; and 0.2 mL of the diluted bacterial solution was spread on an oxygen-free GAM solid medium (supplemented with 5% (v/v) horse serum, Beijing Solarbio Science & Technology Co., Ltd., S9050). Co-culture with pathogenic bacteria: 3 sterilized Oxford cups were placed on each of the spread plates of pathogenic bacteria, and 0.2 mL of each strain fermentation broth was added to the Oxford cups. The plates were placed into anaerobic culture boxes and cultured in an upright position for 24 h, and the sizes of the zones of inhibition were measured using a vernier caliper. The experimental results are shown in FIG. 3. It can be seen that *Parabacteroides distasonis* Pdist-1 has the ability to inhibit *Pseudomonas aeruginosa, Salmonella* Paratyphi B, *Yersinia enterocolitica, Staphylococcus aureus,* and *Vibrio parahaemolyticus* (A); *Limosilactobacillus fermentum* Lferm-1 has the ability to inhibit *Salmonella* Paratyphi B, *Yersinia enterocolitica, Staphylococcus aureus,* and *Clostridioides difficile* (B); *Lactobacillus salivarius* Lsali-1 has the ability to inhibit *Pseudomonas aeruginosa, Shigella, Salmonella* Paratyphi B, *Yersinia enterocolitica, Vibrio parahaemolyticus, Staphylococcus aureus,* and *Clostridioides difficile* (C); *Enterococcus avium* Eaviu-1 has the ability to inhibit *Pseudomonas aeruginosa* and *Clostridioides difficile*, and has a relatively strong ability to inhibit *Clostridioides difficile* (D); and *Bifidobacterium bifidum* Bbifi-1 has the ability to inhibit *Pseudomonas aeruginosa, Shigella, Escherichia coli, Salmonella* Paratyphi B, *Yersinia enterocolitica, Vibrio parahaemolyticus,* and *Clostridioides difficile* (E).

### Example 7: In Vitro Barrier Repair Test

Caco-2 cells (purchased from Shangcheng BeNa Culture Collection, BNCC No. 350769) were seeded into a Transwell plate (a permeable cell culture chamber): Caco-2 adherent cells were digested with a trypsin solution preheated at 37 °C. Caco-2 cells were seeded in a 24-well Transwell plate with a DMEM medium containing 10% (v/v) FBS and 1% (w/v) PS (DMEM medium, purchased from Gibco, Cat. No. C11995500BT; FBS, purchased from Gibco, Cat. No. 16000-044; PS, a penicillin-streptomycin mixed solution, purchased from Gibco, Cat. No. 15140-122) at a seeding density of 1.1 × 10⁵ cells/well, and the plate was cultured and left to stand with 5% CO₂ at 37 °C for 21 d. Strain culture: 200 µL of *Lactobacillus salivarius* Lsali-1 bacterial solution and *Enterococcus avium* Eaviu-1 bacterial solution were each taken from the bacterial preservation tube and added to 5 mL of a two-component mixed medium (the relevant reagents were deoxygenated in advance), and the bacteria were anaerobically cultured in an electric thermostatic incubator at 37 °C for 24 h. The bacteria were passaged and inoculated once, and then anaerobically cultured for 8 h. 1 mL of the bacterial solution was taken and centrifuged at 12,000 rpm/min for 3 min. The strain was diluted to 10⁷ CFU/mL with a DMEM medium containing 10% (v/v) FBS for later use. *Lactobacillus rhamnosus* GG (LGG, CICC 6141, China Center of Industrial Culture Collection) at the same concentration was used as a positive control. Effect of *Lactobacillus salivarius* Lsali-1 and *Enterococcus avium* Eaviu-1 on intestinal epithelial barrier function in Caco-2 cell model: for each bacterium, 4 groups were set, i.e., a normal control group, a model group, a positive control group, and a bacterial group (a *Lactobacillus salivarius* Lsali-1 group and an *Enterococcus avium* Eaviu-1 group). The barrier damage model was constructed using inflammatory factors IFN-γ (Pepro Tech, AF-300-02) and TNF-α (Pepro Tech, 300-01A) as the model group. After being cultured for 21 d, the Caco-2 cells were allowed to differentiate to form a dense monolayer of cells, and the old medium in the lower chamber was pipetted off. 800 µL of DMEM medium was added to the lower chamber of the normal control group, and 800 µL of IFN-γ solutions at a concentration of 10 ng/mL were added to the lower chambers of the model group, the positive control group, and the bacterial groups. After the plates were placed in a 5% carbon dioxide incubator and left to stand for culture at 37 °C for 22 h, the solutions in the upper chambers and the lower chambers were pipetted off. In the normal control group, 200 µL of DMEM medium was added to the upper chamber, and 800 µL of DMEM medium was added to the lower chamber. In the model group, 200 µL of DMEM medium was added to the upper chamber. In the positive control group, 200 µL of positive bacterial solution was added to the upper chamber. In the bacterial groups, 200 µL of *Lactobacillus salivarius* Lsali-1 bacterial solution and *Enterococcus avium* Eaviu-1 bacterial solution were added to the upper chambers, respectively. In the model group, the positive control group, and the bacterial groups, 800 µL of TNF-α solutions at a concentration of 50 ng/mL were added to the lower chambers. After the plates were placed in a 5% carbon dioxide incubator and left to stand for culture at 37 °C for 5 h, the transepithelial electrical resistance (TEER) value of the cell monolayer in each group was measured.

The results are shown in FIG. 4. It can be seen that compared with the model group, the positive control group LGG could significantly increase the TEER value, indicating that LGG has a significant repair effect on the cell barrier damage. Similarly, compared with the model group, *Lactobacillus salivarius* Lsali-1 (A) and *Enterococcus avium* Eaviu-1 (B) could also significantly increase the TEER values, and had similar or comparable effects to that of LGG. The results indicate that *Lactobacillus salivarius* Lsali-1 and *Enterococcus avium* Eaviu-1 can effectively alleviate the barrier dysfunction caused by inflammatory factors (e.g., IFN-γ and TNF-α).

### Example 8: In Vitro Cell Inflammation Inhibition Test

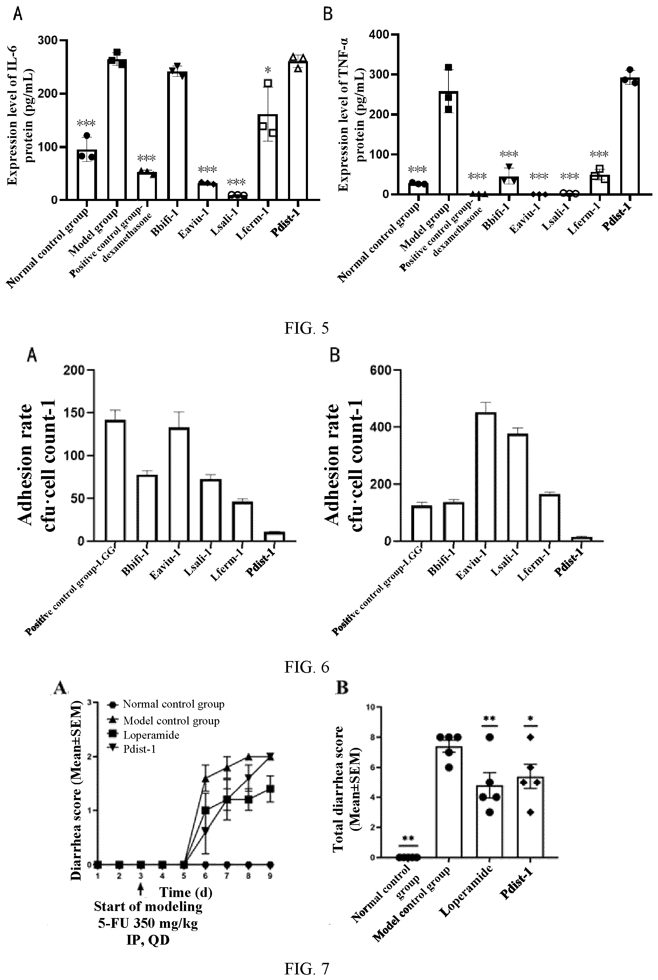

Polarization of THP-1 cells: THP-1 cells were seeded into a 96-well plate at a seeding density of 1 × 10⁵ cells/well using an RPMI-1640 medium (Thermo Fisher, C11875500BT) containing 10% (v/v) FBS and PMA (phorbol 12-myristate 13-acetate, Sigma-Aldrich Company, P1585) at a final concentration of 100 ng/mL. The 96-well plate was placed in a 5% CO ₂ incubator and cultured at 37 °C for 24 h to polarize the cells into mature macrophages. Strain culture: 200 µL of each of the test bacterial solutions of *Parabacteroides distasonis* Pdist-1, *Limosilactobacillus fermentum* Lferm-1, *Lactobacillus salivarius* Lsali-1, *Enterococcus avium* Eaviu-1, and *Bifidobacterium bifidum* Bbifi-1 was taken from the bacterial preservation tube and inoculated into 5 mL of a two-component mixed medium, and then the bacteria were anaerobically cultured at 37 °C for 24 h. After one transfer, the bacteria were anaerobically cultured for 8 h. 1 mL of the test bacterial solution was taken and centrifuged at 5,000 rpm/min for 15 min. The aforementioned strains were sequentially diluted with an RPMI-1640 medium containing 10% (v/v) FBS to 5 × 10⁷ CFU/mL, 2 × 10⁶ CFU/mL, 5 × 10⁷ CFU/mL, 5 × 10⁷ CFU/mL, and 5 × 10⁷ CFU/mL for later use. Effect on the expression of TNF-α and IL-6 in THP-1 cells: after THP-1 mature cells were cultured, the medium in the normal control group (without bacterial or drug treatment) was replaced with an RPMI-1640 medium containing 10% (v/v) FBS; the media in the model group, the positive control group (treated with dexamethasone), and the test groups (treated with the strains) were replaced with RPMI-1640 media containing 10% (v/v) FBS, LPS (Sigma-Aldrich Company, L3024) at a final concentration of 100 ng/mL, and IFN-γ (PeproTech, AF-300-02) at a final concentration of 20 ng/mL, respectively; and the modeling of inflammatory macrophages was performed. The plate of each group was placed in a 5% CO₂ incubator and cultured at 37 °C for 24 h. The media were pipetted off; 100 µL of RPMI-1640 media containing 10% (v/v) FBS were added to the normal control group and the model group, respectively; 100 µL of an RPMI-1640 medium containing 10% (v/v) FBS and dexamethasone (purchased from Sigma-Aldrich Company, D4902-25) at a final concentration of 25 µg/mL was added to the positive control group; and 100 µL of the previously prepared test bacterial solutions were added to the test groups, respectively. The plates were placed in a 5% CO₂ incubator and cultured at 37 °C for 24 h. 80 µL of cell culture solution was taken from each group and centrifuged at 4 °C and 5,000 rpm/min for 15 min. The supernatant was collected, and the TNF-α content was assayed using a Human TNF-α (tumor necrosis factor alpha) ELISA kit (purchased from Wuhan Elabscience Biotechnology Co., Ltd., E-EL-H0109c), and the IL-6 content was assayed using a Human IL-6 (interleukin 6) ELISA kit (purchased from Wuhan Elabscience Biotechnology Co., Ltd., E-EL-H6156). The experimental results are shown in FIG. 5. It can be seen that the expression of IL-6 and TNF-α in the cells of the model control group was significantly higher than that of the normal control group (P < 0.01); the positive control dexamethasone group could significantly inhibit the expression of the pro-inflammatory factors IL-6 and TNF-α in the THP-1 cells (P < 0.01); compared with the model group, the expression of the pro-inflammatory factor IL-6 could be significantly reduced by *Limosilactobacillus fermentum* Lferm-1, *Lactobacillus salivarius* Lsali-1, and *Enterococcus avium* Eaviu-1, and the expression of the pro-inflammatory factor TNF-α could be significantly reduced by *Limosilactobacillus fermentum* Lferm-1, *Lactobacillus salivarius* Lsali-1, *Enterococcus avium* Eaviu-1, and *Bifidobacterium bifidum* Bbifi-1 (P < 0.05), indicating that these strains have antiinflammatory effects *in vitro.*

### Example 9: Test of Adhesion Ability to Caco-2 Cells

Strain culture: 200 µL of *Bifidobacterium bifidum* Bbifi-1 bacterial solution, *Enterococcus avium* Eaviu-1 bacterial solution, *Lactobacillus salivarius* Lsali-1 bacterial solution, *Limosilactobacillus fermentum* Lferm-1 bacterial solution, and *Parabacteroides distasonis* Pdist-1 bacterial solution were each inoculated into 5 mL of a two-component mixed medium, and the bacteria were anaerobically cultured at 37 °C until the late logarithmic growth phase was reached. The cultured bacterial solution was centrifuged and washed twice with sterile PBS (Wuhan Boster Biological Technology Co., Ltd., PYG0021), and the strain was diluted to 5 × 10⁸ CFU/mL with a DMEM medium (Thermo Fisher Scientific (China) Co., Ltd., C11995500BT) containing 10% (v/v) FBS (Thermo Fisher Scientific (China) Co., Ltd., SH30084.03) for later use. *Lactobacillus rhamnosus* GG (LGG, CICC 6141, China Center of Industrial Culture Collection) at the same concentration was used as a positive control group. 100 µL of the diluted bacterial suspension was added to a 96-well cell culture plate containing Caco-2 cells. After the addition, the 96-well cell culture plate was placed in a horizontal centrifuge and centrifuged at 1,000 g for 1 min. The wells corresponding to each bacterial solution were divided into two groups, and the two groups were incubated for 30 min and 2 h, respectively. The plate was washed twice with sterile PBS to wash away non-adherent strains. After washing, 50 µL of a trypsin solution (Labgic Technology Co., Ltd., BL501A) was added to each well, and the cells were digested in an incubator at 37 °C. After the Caco-2 cells were digested and became spherical, 150 µL of a DMEM medium containing 10% (v/v) FBS was added to each well, and the mixture was pipetted repeatedly for about 1 min. After the cells and strains were digested and isolated as confirmed by microscopy, 20 µL of the above mixed solution was pipetted and serially 10-fold diluted with 0.1% Tween 80-PBS in the 96-well plate, and an appropriate dilution gradient was selected to pour the dissolved three-component mixed solid medium. The cells were cultured at 37 °C for 48 h and then counted. The results are shown in FIG. 6, where FIG. 6A shows the adhesion effect of each strain when the incubation time was 30 min, and shows that the adhesion ability of *Enterococcus avium* Eaviu-1 was comparable to that of the positive control group LGG; FIG. 6B shows the adhesion effect of each strain when the incubation time was 2 h, and shows that the adhesion abilities of *Bifidobacterium bifidum* Bbifi-1 and *Limosilactobacillus fermentum* Lferm-1 were comparable to that of the positive control group LGG, the adhesion abilities of *Lactobacillus salivarius* Lsali-1 and *Enterococcus avium* Eaviu-1 were superior to that of the positive control group LGG, and *Parabacteroides distasonis* Pdist-1 had certain adhesion, indicating that the 5 strains have the potential to colonize the intestinal tract.

### Example 10: Therapeutic Effect on Mouse Model with 5-Fluorouracil (5-FU)-Induced Diarrhea

Preparation of lyoprotectant for *Parabacteroides distasonis* Pdist-1: solution A: 6 g of sucrose, 6 g of trehalose, 2 g of xylitol, 2 g of sorbitol, and 44 g of purified water; solution B: 5 g of sodium glutamate and 15 g of purified water, sterilized at 115 °C for 20 min; solution C: 4 g of sodium ascorbate and 16 g of purified water. These solutions were filtered and sterilized for later use. Preparation of lyoprotectants for *Limosilactobacillus fermentum* Lferm-1, *Lactobacillus salivarius* Lsali-1, and *Enterococcus avium* Eaviu-1: solution A: 8 g of sucrose, 8 g of trehalose, and 44 g of purified water, sterilized at 115 °C for 30 min; solution B: 2 g of sodium glutamate, 2 g of arginine hydrochloride, and 16 g of purified water, sterilized at 115 °C for 30 min; solution C: 4 g of sodium ascorbate and 16 g of purified water. Preparation of lyoprotectant for *Bifidobacterium bifidum* Bbifi-1: solution A: 6 g of sucrose, 6 g of trehalose, 2 g of xylitol, 2 g of sorbitol, and 44 g of purified water, sterilized at 115 °C for 30 min; solution B: 2 g of arginine hydrochloride, 2 g of sodium glutamate, and 16 g of purified water, sterilized at 115 °C for 30 min; solution C: 4 g of sodium ascorbate and 16 g of purified water. These solutions were filtered and sterilized for later use.

The lyoprotectant for each strain was mixed in a volume ratio of solution A: solution B:solution C = 6:2:2 when used.

Preparation of bacterial powder: the 5 strains obtained in Example 1 were each inoculated into the corresponding bacterial powder preparation medium, and anaerobically cultured at 37 °C and 90 rpm for 16-24 h to obtain a primary seed solution. Subsequently, the primary seed solution was transferred to the corresponding bacterial powder preparation medium, and anaerobically cultured at 37 °C and 90 rpm for 10-15 h to obtain a secondary seed solution. The secondary seed solution was pumped into a fermenter containing the corresponding bacterial powder preparation medium using a peristaltic pump, and subjected to fermentation culture. After the fermentation was stopped, the bacteria were collected by centrifugation. The lyoprotectant was added according to a weight ratio of the bacterial sludge to the lyoprotectant of 1:1 to 1:2, and the emulsified bacterial sludge was well mixed. The mixture was lyophilized and pulverized to obtain the bacterial powder. Before administration to the animals, the bacterial powder containing 1 × 10⁹ CFU live bacteria was prepared into a bacterial suspension using 0.2 mL of normal saline. Test animals: SPF-grade male Balb/c mice, weighing 18-22 g, purchased from Beijing Vital River Laboratory Animal Technology Co., Ltd., housed in an SPF-grade animal room. The mice were randomly divided into groups according to the initial weight, with 5 mice in each group. For each of *Parabacteroides distasonis* Pdist-1, *Limosilactobacillus fermentum* Lferm-1, *Enterococcus avium* Eaviu-1, and *Bifidobacterium bifidum* Bbifi-1, 4 groups were set, i.e., a normal control group, a model control group, a positive control loperamide group, and a test strain group. For *Lactobacillus salivarius* Lsali-1, 4 groups were set, i.e., a normal control group, a model control group, a control strain Lsali-3 group of the same species (Lsali-3 was another *Lactobacillus salivarius* strain isolated by the same method as in Example 1), and a test strain Lsali-1 group. Test design: a 5-FU solution (5-fluorouracil, purchased from Tianjin Pharma Heping (Tianjin) Pharmaceutical Co., Ltd., specification: 10 mL/vial, 0.25 g/10 mL) was used to induce chemotherapy-related diarrhea models in mice. Except that the normal control group was intraperitoneally injected with normal saline, other groups were treated with 5-FU single intraperitoneal injection for modeling, with a molding dose of 350 mg/kg body weight. The administration mode for all groups was intragastric administration. The normal control group and the model control group were intragastrically given the lyoprotectants. The positive control group was intragastrically given loperamide (purchased from Xian Janssen Pharmaceutical Ltd.) at 20 mg/kg body weight. The test strain groups were intragastrically given the bacterial suspensions of the test strains at a dose of 1 × 10⁹ CFU/mouse. The overall experimental period was 9 days, which were recorded as D1-D9. Modeling treatment was performed on D3. The normal control group, the model control group, and the test strain groups were intragastrically given the test substances for consecutive days from D1 to D5, and the positive control group was intragastrically given loperamide for consecutive days from D1 to D9. After the end of the administration on D5, the mice were observed for 4 consecutive days. The specific experimental groups and administration regimens are shown in Table 4 below.

**Table 4. Experimental groups and administration regimens for treatment of mice with 5-FU-induced diarrhea**

| **Group** | **Number** | **Modeling agent** | **Modeling dose** | **Test substance** | **Volume of administration** | **Dose of administration** | **Days of administration** |
|---|---|---|---|---|---|---|---|
| Normal control group | 5 | Normal saline | / | Lyoprotectant | 0.2 mL/mouse | / | 5 d |
| Model control group | 5 | 5-FU | 350 mg/kg | Lyoprotectant | 0.2 mL/mouse | / | 5 d |
| Loperamide | 5 | 5-FU | 350 mg/kg | Loperamide | 10 mL/kg body weight | 20 mg/kg body weight | 9 d |
| Strain group | 5 | 5-FU | 350 mg/kg | Each test strain | 0.2 mL/mouse | 1 × 10⁹ CFU/mouse | 5 d |

| | | | | | | | |
|---|---|---|---|---|---|---|---|
| Note: 5-FU indicates 5-fluorouracil; CFU indicates colony forming unit; d indicates days | | | | | | | |

Diarrhea observation and scoring: the mice were placed in mouse cages with clean filter paper at the bottom, with 1 mouse per cage. Hard feces and normal feces were considered as 0 points; mild diarrhea was characterized by slightly wet feces or soft feces, which was considered as 1 point; moderate diarrhea was characterized by wet feces, shapeless feces, and perianal uncleanliness, which was considered as 2 points; and severe diarrhea was characterized by loose feces and severe perianal uncleanliness, which was considered as 3 points. During the experiment, the feces of the mice were observed and scored daily. The total diarrhea score is the sum of daily diarrhea scores.

Body weight measurement and rate of change in body weight: during the experiment, the mice were weighed daily, and the rate of change in body weight was calculated as follows: rate of change in body weight = (measured body weight - initial body weight)/initial body weight × 100%.

At the end of the experiment, all mice were dissected, and the entire cecum together with the colorectum was separated along with the anus. The colorectum length of the mice was measured using a ruler with the end of the cecum as the zero point and the end of the rectum as the endpoint.

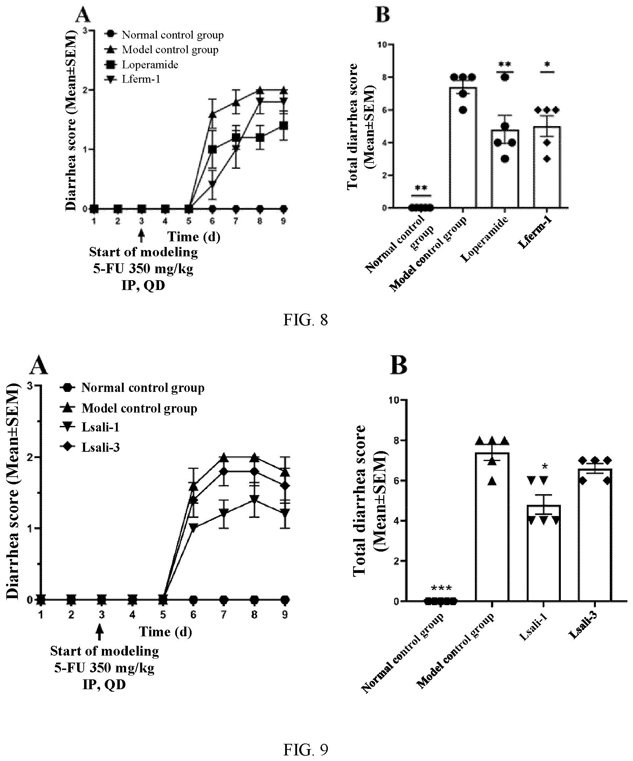

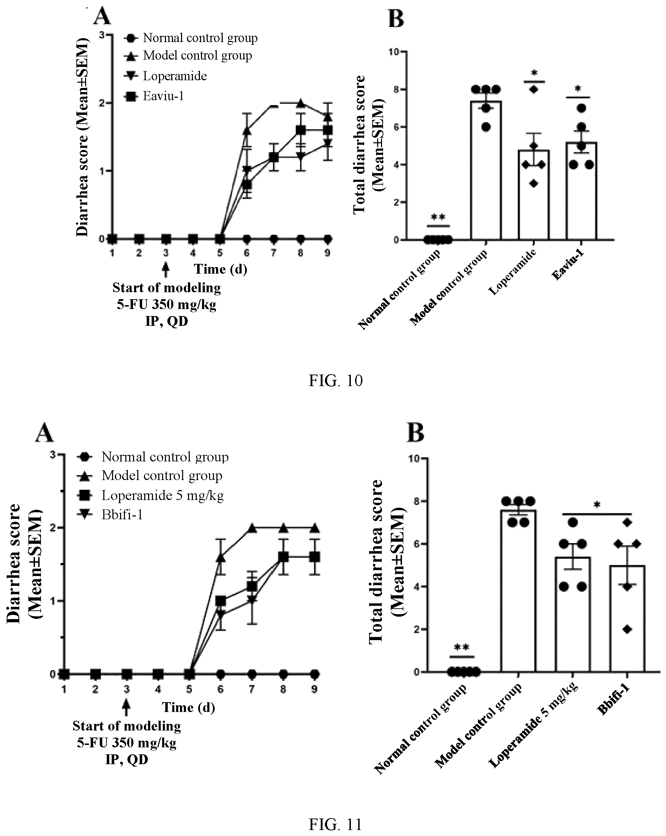

The results of the diarrhea experiment are shown in FIGs. 7-11. It can be seen that compared with the model control group, the five strains each exhibited a significant improvement effect on the 5-FU-induced diarrhea, and the total diarrhea scores were significantly reduced.

In order to compare the differences in efficacy between *Bifidobacterium bifidum* Bbifi-1 and *Bifidobacterium bifidum* standard strain DSM20456 (purchased from German Collection of Microorganisms and Cell Cultures), a CID model was constructed as described above. The previous data show that stable diarrhea could be observed 4 days after the CID modeling, i.e., D7. Therefore, the overall experimental period of this experiment was 7 days, which were recorded as D1-D7. On D3, except that the normal control group was intraperitoneally injected with normal saline, other groups were treated with 5-FU single intraperitoneal injection for modeling, with a molding dose of 350 mg/kg body weight, and the Bbifi-1 group and the DSM20456 group were intragastrically given 1 × 10⁹ CFU daily. During the experiment, the feces of the mice were observed and scored daily, and the scoring criteria were kept consistent with the previous criteria. The specific schemes are shown in Table 5 below.

**Table 5. Experimental groups and administration regimens for treatment of mice with 5-FU-induced diarrhea**

| **Group** | **Number** | **Modeling agent** | **Modeling dose** | **Test substance** | **Volume of administration** | **Dose of administration** | **Days of administration** |
|---|---|---|---|---|---|---|---|
| Normal control group | 5 | Normal saline | / | Normal saline | 0.2 mL/mouse | / | 7 d |
| Model control group | 5 | 5-FU | 350 mg/kg | Normal saline | 0.2 mL/mouse | / | 7 d |
| Loperamide group | 5 | 5-FU | 350 mg/kg | Loperamide | 20 mL/kg | 20 mg/kg | 7 d |
| Bbifi-1 group | 5 | 5-FU | 350 mg/kg | Bbifi-1 | 0.2 mL/mouse | 1 × 10⁹ CFU/mouse | 7 d |
| DSM20456 | 5 | 5-FU | 350 mg/kg | DSM20456 | 0.2 mL/mouse | 1 × 10⁹ | 7 d |
| group | CFU/mouse | | | | | | |

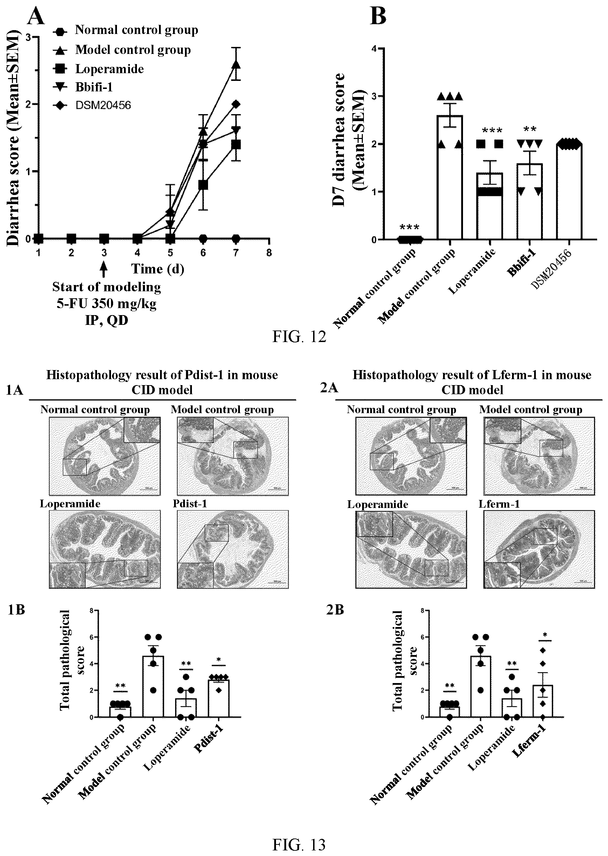

The experimental results are shown in FIG. 12. Compared with the model control group, the effect of Bbifi-1 in alleviating 5-FU-induced diarrhea was superior to that of the standard strain DSM20456.

### Effect on Colon Injury

After the animal experiment, the middle colon of the mice was collected and fixed in 4% paraformaldehyde for 24 h. The fixed colon tissues were sequentially dehydrated, transparentized, waxed, and embedded. The embedded paraffin blocks of colon tissues were sectioned at a thickness of 5 µm, followed by spreading and baking, and the dried sections were subjected to conventional HE staining. The pathological changes were observed under an optical microscope. Pathological scoring was performed according to Table 6 below, and the total pathological scores (the sum of the scores of all indices) were calculated.

**Table 6. Pathological scoring indices and description of colon tissues**

| **Index** | **Grade** | **Description** |
|---|---|---|
| Epithelial injury | 0 | No injury, no significant degeneration, necrosis, or other lesions of epithelial cells or goblet cells, with intact mucosal structure |
| | 1 | Mild injury, degeneration or necrosis of a small number of cells (≤ 20%) in the field of view, with intact mucosal structure |
| | 2 | Moderate injury, significant cell degeneration or necrosis (≤ 40%) in the field of view, with substantially intact mucosal structure |
| | 3 | Severe injury, degeneration or necrosis of a large number of cells (≥ 40%) in the field of view, absence of mucosal structure, and significant narrowing |
| Inflammatory cells Infiltration | 0 | No inflammatory cell infiltration |
| | 1 | Mild infiltration, a small number of inflammatory cells or inflammatory foci in the mucosa, with a relatively small inflammatory focus area, or infiltration into the lamina propria |
| | 2 | Moderate infiltration, a small number of inflammatory cells or inflammatory foci in the mucosa, with a moderate inflammatory focus area, or infiltration into the muscularis mucosae and submucosa |
| | 3 | Severe infiltration, a large number of inflammatory cells or inflammatory foci in the mucosa, with a relatively large inflammatory focus area, or transmural infiltration involving the muscularis propria |
| Thickness of muscularis propria | 0 | Normal |
| | 1 | Reduced |
| Crypt abscess | 0 | Absence of abscess in the crypt |
| | 1 | Presence of abscess in the crypt |
| Number of goblet cells | 0 | Normal goblet cell number |
| | 1 | Loss of goblet cells, reduced number |

As can be seen from panels 1A and 2A in FIG. 13, in the normal control group, the colon structure was intact, and the mucosal layer, submucosa, muscularis propria, and serosal layer were clearly visible; in the lamina propria of the colon of the model control group, necrosis and dissolution of intestinal glands, a reduction in goblet cells, lymphocyte infiltration, loose connective tissue of the submucosa, and vasodilation were observed; in the loperamide group, the colon structure was relatively intact, mucosal epithelial cells showed no significant necrosis, the number of goblet cells was significantly increased, and only a small amount of lymphocyte infiltration was observed in the lamina propria; in the Pdist-1 group, the mucosal layer structure of the colon was relatively intact, mucosal epithelial cells showed no significant necrosis, and the number of goblet cells was significantly increased as compared to the model control group; in the Lferm-1 group, the colon tissue showed a small amount of mucosal epithelial cell necrosis accompanied by a small amount of lymphocyte infiltration, a small number of intestinal glands showed dilation, the number of goblet cells was slightly reduced, and mild edema was observed in the submucosa accompanied by a small amount of lymphocyte infiltration. As can be seen from panels 1B and 2B in FIG. 13, significant colon injury occurred in the mice after 5-FU induction, and the total pathological score of the model control group was significantly higher than that of the normal control group (*P* < 0.01). After administration, *Parabacteroides distasonis* Pdist-1 could significantly reduce the total pathological score of the colon (*P* < 0.05) and ameliorate colon injury. *Limosilactobacillus fermentum* Lferm-1 could also significantly reduce the total pathological score of the colon (*P* < 0.05) and ameliorate the degree of colon injury.

### Improvement in Relative mRNA Transcription Level of IL-1β, TNF-α, and AQP8 in Mouse Colon

After the animal experiment, the middle colon of the mice was collected and stored in a refrigerator at -80 °C. The total RNA was extracted from the colon tissues of the mice in each group according to the instruction of the reagent (ThermoFisher Scientific, Cat. No. 15596026), and reverse-transcribed into cDNA. The cDNA was then stored at -20 °C for later use. The relative mRNA transcription levels of pro-inflammatory factors *IL-1β* and *TNF-α,* as well as aquaporin 8 (*AQP8*) genes in mouse colon in each group, were detected by qRT-PCR (the primer sequences are shown in Table 7). Reaction program: 95 °C for 3 min, 95 °C for 20 s, 60 °C for 45 s, and 72 °C for 20 s, 39 cycles in total. Analysis was performed using the 2^{-ΔΔCT} method, and the data were subjected to significance analysis using the SPSS 24.0 statistical software.

**Table 7. qRT-PCR primer information**

| Gene | Primer | |
|---|---|---|
| Interleukin 1 beta (*IL-1β*) | Forward (SEQ ID NO.1): | 5'- AGTTGACGGACCCCAAAAG -3' |
| | Reverse (SEQ ID NO.2): | 5'- AGCTGGATGCTCTCATCAGG -3' |
| Tumor necrosis factor (*TNF-α*) | Forward (SEQ ID NO.3): | 5'-CTGTAGCCCACGTCGTAGC-3' |
| | Reverse (SEQ ID NO.4): | 5'-TTGAGATCCATGCCGTTG-3' |
| Aquaporins8 (*AQP8*) | Forward (SEQ ID NO.5): | 5'-GGAACATCAGCGGTGGACACTTC-3' |
| | Reverse (SEQ ID NO.6): | 5'-GGGAATTAGCATGGTCTTGAGG-3' |
| 18S rRNA | Forward (SEQ ID NO.7): | 5'-CTCAACACGGGAAACCTCAC-3' |
| | Reverse (SEQ ID NO.8): | 5'-CGCTCCACCAACTAAGAACG-3' |

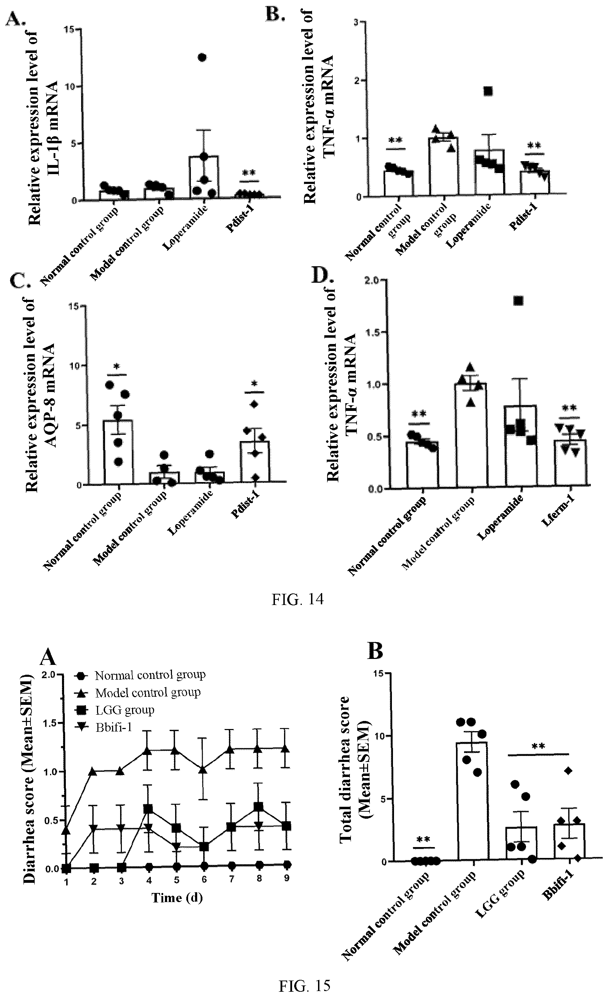

The results are shown in FIG. 14, and it can be seen that in the model control group, the relative mRNA transcription levels of *IL-1β* and *TNF-α* were significantly increased, and the relative mRNA transcription level of *AQP8* was significantly reduced, as compared with the normal control group. Compared with the model control group, the administration of Pdist-1 could significantly reduce the relative mRNA transcription levels of *IL-1β* and *TNF-α* (A and B), and significantly increase the relative mRNA transcription level of *AQP8* (C); and the administration of Lferm-1 could significantly reduce the relative mRNA transcription level of *TNF-α* (*D*).

### Example 11: Therapeutic Effect of Bifidobacterium bifidum Bbifi-1 on Radiation Enteritis Model Mice

The lyoprotectant and the bacterial powder were prepared by the same method as in Example 10. Test animals: 20 SPF-grade male C57BL/6J mice, weighing 20-25 g, purchased from Beijing Vital River Laboratory Animal Technology Co., Ltd., housed in an SPF-grade animal room. Test design: a radiation enteritis mouse model was induced by using abdominal X-ray irradiation. The mice were randomly divided into 4 groups according to the initial body weight, i.e., a normal control group, a model control group, a positive control group, and a *Bifidobacterium bifidum* Bbifi-1 group, with 5 mice in each group. Except for the normal control group, which was not irradiated, all other groups were subjected to single abdominal X-ray irradiation for modeling, with a modeling dose of 11.5 Gy. The administration mode for all the groups was intragastric administration. The normal control group and the model control group were intragastrically given normal saline; the positive control group was intragastrically given 1 × 10⁹ CFU of *Lactobacillus rhamnosus* GG (LGG, purchased from Shaanxi Zelang Biotechnology Co., Ltd.); the *Bifidobacterium bifidum* Bbifi-1 group was intragastrically given 1 × 10⁹ CFU of *Bifidobacterium bifidum* Bbifi-1. The overall experimental period was 13 days, which were recorded as D-2 to D10. The mice were intragastrically given the test substances for 12 consecutive days from D-2 to D9, and treated with single abdominal X-ray irradiation on D0 (day 3).

Diarrhea observation and scoring: the mice were placed in mouse cages with clean filter paper at the bottom, with 1 mouse per cage. Hard feces and normal feces were considered as 0 points; mild diarrhea was characterized by slightly wet feces or soft feces, which was considered as 1 point; moderate diarrhea was characterized by wet feces, shapeless feces, and perianal uncleanliness, which was considered as 2 points; and severe diarrhea was characterized by loose feces and severe perianal uncleanliness, which was considered as 3 points. During the experiment, the feces of the mice were observed and scored daily. The total diarrhea score is the sum of daily diarrhea scores. On D10 (day 13), the experimental animals were euthanized by pentobarbital sodium anesthesia, and then subjected to gross anatomy and anatomical observation. The specific experimental groups and administration regimens are shown in Table 8 below.

**Table 8. Experimental groups and administration regimens for treatment of mice with X-ray-induced diarrhea by Bifidobacterium bifidum Bbifi-1**

| **Group** | **Number** | **Modeling mode** | **Modeling dose** | **Test substance** | **Volume of administration** | **Dose of administration** | **Days of administration** |
|---|---|---|---|---|---|---|---|
| Normal control group | 5 | Normal saline | / | Lyoprotectant | 0.2 mL/mouse | / | 12 d |
| Model control group | 5 | Abdominal X-ray irradiation | 11.5 Gy | Lyoprotectant | 0.2 mL/mouse | / | 12 d |
| Positive control group | 5 | Abdominal X-ray irradiation | 11.5 Gy | *Lactobacillus rhamnosus* GG | 0.2 mL/mouse | 1 × 10⁹ CFU/mouse | 12 d |
| *Bifidobacterium bifidum* Bbifi-1 group | 5 | Abdominal X-ray irradiation | 11.5 Gy | *Bifidobacterium bifidum* Bbifi-1 | 0.2 mL/mouse | 1 × 10⁹ CFU/mouse | 12 d |

| | | | | | | | |
|---|---|---|---|---|---|---|---|
| Note: CFU indicates colony forming unit; d indicates days | | | | | | | |

The test results are shown in FIG. 15, where panel A shows the diarrhea score curve of each group from day 1 to day 12, and panel B shows the total diarrhea score of each group. It can be seen that compared with the model control group, *Bifidobacterium bifidum* Bbifi-1 exhibited a significant improvement effect on the X-ray irradiation-induced diarrhea, which was comparable to that of the positive control group LGG (A); LGG and *Bifidobacterium bifidum* Bbifi-1 could significantly reduce the total diarrhea scores (P < 0.05, B). The above results indicate that *Bifidobacterium bifidum* Bbifi-1 has a significant improvement effect on the X-ray irradiation-induced diarrhea of the mice.

### Example 12: Test of Co-Culture Characteristics Among Strains

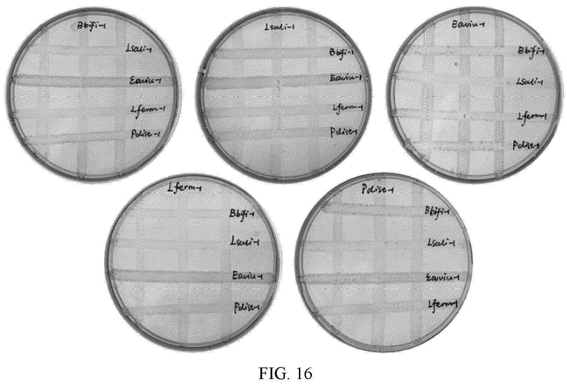

The 5 strains obtained in Example 1 were each cultured for activation until the late logarithmic growth phase was reached. The bacterial solution of one of the strains was streaked three times in parallel on a BF839 solid medium using a disposable sterile cotton swab, and then the bacterial solutions of the other four strains were streaked once in parallel in a direction perpendicular to the first streak. After the streaked bacterial solutions were dried, the bacteria were anaerobically cultured for 48 h until the bacterial solutions had significant marks. The interaction relationships among the five strains are shown in FIG. 16. It can be seen that there were no breakpoints at the intersections of the strains, indicating that no growth inhibition occurs among the strains.

It can be known from the general technical knowledge that the present disclosure can be implemented by other embodiments without departing from the spirit or essential features thereof. Therefore, the embodiments disclosed above are illustrative in all aspects and are not the only ones. All modifications within the scope of the present disclosure or within the scope equivalent to the present disclosure are encompassed in the present disclosure.

## Claims

1. A probiotic composition, wherein an active ingredient in the probiotic composition comprises any one or a combination of two or more of the following: *Bifidobacterium bifidum* with the microbial deposit number of CCTCC NO: M 2023349 or a pure culture thereof, *Enterococcus avium* with the microbial deposit number of CCTCC NO: M 2023350 or a pure culture thereof, *Lactobacillus salivarius* with the microbial deposit number of CCTCC NO: M 2023348 or a pure culture thereof, *Limosilactobacillus fermentum* with the microbial deposit number of CCTCC NO: M 2023352 or a pure culture thereof, and *Parabacteroides distasonis* with the microbial deposit number of CCTCC NO: M 20222033 or a pure culture thereof.

2. The probiotic composition according to claim 1, further comprising a lyoprotectant, a food material, a pharmaceutically acceptable carrier, and/or a pharmaceutically acceptable excipient.

3. An isolated *Parabacteroides distasonis* strain or a pure culture thereof, wherein the *Parabacteroides distasonis* strain has a microbial microbial deposit number of CCTCC NO: M20222033.

4. An isolated *Limosilactobacillus fermentum* strain or a pure culture thereof, wherein the *Limosilactobacillus fermentum* strain has a microbial microbial deposit number of CCTCC NO: M2023352.

5. An isolated *Lactobacillus salivarius* strain or a pure culture thereof, wherein the *Lactobacillus salivarius* strain has a microbial microbial deposit number of CCTCC NO: M2023348.

6. An isolated *Enterococcus avium* strain or a pure culture thereof, wherein the *Enterococcus avium* strain has a microbial microbial deposit number of CCTCC NO: M2023350.

7. An isolated *Bifidobacterium bifidum* strain or a pure culture thereof, wherein the *Bifidobacterium bifidum* strain has a microbial microbial deposit number of CCTCC NO: M2023349.

8. A method for preventing, treating, or slowing an intestinal disease, comprising administering to a subject a therapeutically effective amount of the probiotic composition according to claim 1 or 2, or the strain or the pure culture thereof according to any one of claims 3-7.

9. The method according to claim 8, wherein the subject is a mammal; further preferably, the subject is a human.

10. The method according to claim 8, wherein the probiotic composition according to claim 1 or 2, or the strain or the pure culture thereof according to any one of claims 3-7, inhibits intestinal pathogenic bacteria in the subject, ameliorates colon injury of the subject, suppresses intestinal inflammation of the subject, repairs intestinal barrier of the subject, or alleviates symptoms of diarrhea in the subject.

11. The method according to claim 8, wherein the intestinal disease is selected from an infection caused by an intestinal pathogenic bacterium, diarrhea, and radiation enteritis; preferably, the intestinal pathogenic bacterium is selected from one or more of *Pseudomonas aeruginosa, Salmonella* Paratyphi B, *Yersinia enterocolitica, Staphylococcus aureus, Vibrio parahaemolyticus, Clostridioides difficile*, *Shigella,* and *Escherichia coli*; preferably, the diarrhea is diarrhea caused by an anti-tumor drug, and further preferably, the diarrhea caused by an anti-tumor drug is diarrhea caused by a chemotherapeutic drug.

12. The method according to claim 11, wherein the diarrhea caused by a chemotherapeutic drug is diarrhea caused by one or more drugs selected from the following: epirubicin, actinomycin D, doxorubicin, daunorubicin, paclitaxel, docetaxel, albumin-bound paclitaxel, cisplatin, carboplatin, nedaplatin, oxaliplatin, lobaplatin, cyclophosphamide, nitrogen mustard, carmustine, camptothecin, hydroxycamptothecin, topotecan, irinotecan, capecitabine, gemcitabine, methotrexate, 5-fluorouracil, pemetrexed, and cytarabine.
